# EUROPÄISCHE PATENTSCHRIFT

(11) **EP 2 303 165 B1**
(45) Veröffentlichungstag und Bekanntmachung des Hinweises auf die Patenterteilung: **11.11.2015**
(21) Anmeldenummer: 09772188.0
(22) Anmeldetag: 04.07.2009
(51) Int. Cl.: A61B 17/70

(54) **IMPLANTAT ZUR GEGENSEITIGEN ABSTÜTZUNG DER DORNFORTSÄTZE VON WIRBELKÖRPERN**
IMPLANT FOR MUTUALLY SUPPORTING THE SPINOUS PROCESSES OF VERTEBRAL BODIES
IMPLANT POUR LE SOUTIEN MUTUEL DES APOPHYSES ÉPINEUSES DE VERTÈBRES

(30) Priorität: 04.07.2008 DE 102008032685
(43) Veröffentlichungstag der Anmeldung: 06.04.2011
(73) Patentinhaber: Aesculap AG, 78532 Tuttlingen (DE)
(72) Erfinder: MAAS, Allan, 78465 Konstanz (DE); STOERK, Claudia, 78576 Emmingen (DE); HAAS, Alexander, 78166 Donaueschingen (DE)
(74) Vertreter: Hoeger, Stellrecht & Partner Patentanwälte mbB
(86) Internationale Anmeldenummer: PCT/EP2009/004844
(87) Internationale Veröffentlichungsnummer: WO 2010/000488

(56) Entgegenhaltungen:
- WO-A-2006/111174
- WO-A-2007/127689
- DE-U1-202008 009 344
- US-B1- 6 332 883

## Beschreibung

Die Erfindung betrifft ein Implantat zur gegenseitigen Abstützung der Dornfortsätze von zwei benachbarten Wirbelkörpern mit mindestens einer oberen Stützfläche für den Dornfortsatz eines oberen Wirbelkörpers und mindestens einer unteren Stützfläche für den Dornfortsatz eines unteren Wirbelkörpers, deren Abstand voneinander vergrößerbar ist.

Derartige Implantate werden in den Zwischenraum zwischen die Dornfortsätze von benachbarten Wirbelkörpern eingeschoben und dann nach dem Einsetzen so verändert, dass die Stützflächen ihren Abstand voneinander vergrößern, so dass die auf den Stützflächen aufruhenden Dornfortsätze dadurch mehr oder weniger auseinander geschoben werden, beispielsweise zur Stabilisierung der benachbarten Wirbelkörper oder zur Entlastung des Zwischenwirbelraumes, teilweise auch zur Verschwenkung der Wirbelkörper gegeneinander.

Für das Einsetzen ist es wesentlich, dass die Implantate möglichst eine geringe Bauhöhe aufweisen, um durch kleine Zugänge die Implantate positionieren zu können, andererseits muss der Abstand der Stützflächen nach dem Einsetzen vergrößert werden, um die gewünschte Distanz zwischen den Dornfortsätzen erzielen zu können.

Die Merkmale des Oberbegriffs des Anspruchs 1 sind aus US -B1 - 6 332 883 bekannt.

Es ist Aufgabe der Erfindung, ein gattungsgemäßes Implantat so auszubilden, dass bei einem relativ einfachen Aufbau die Implantation und die Verstellung des Abstandes der Stützflächen voneinander möglichst einfach erfolgen kann.

Diese Aufgabe wird durch ein Implantat gemäß den Merkmalen des Anspruchs 1 gelöst.

Es werden also bei dem erfindungsgemäßen Implantat zwei Implantatkomponenten gegeneinander geschoben, und dabei gleiten die Stützarme der jeweils einen Implantatkomponente an den Aufgleitflächen der jeweils anderen Implantatkomponente auf und werden so verschwenkt oder gespreizt, dass dadurch die Stützflächen sich voneinander entfernen, also der Stützabstand zwischen den Dornfortsätzen vergrößert wird. Auf diese Weise kann das Implantat mit geringer Bauhöhe implantiert werden, dabei haben die beiden Implantatkomponenten einen relativ großen Abstand voneinander. Nach der Implantation werden die beiden Implantatkomponenten gegeneinander geschoben, und dies führt zwangsläufig zur Vergrößerung des Abstandes der Stützflächen und damit zum Auseinanderdrücken der an den Stützflächen anliegenden Dornfortsätze.

Günstig ist es, wenn die Stützflächen konkav gekrümmt sind, so dass bei einer Abstützung des Dornfortsatzes im mittleren Teil einer Stützfläche diese mit ihren Randbereichen zu beiden Seiten des Dornfortsatzes ansteigt. Diese Formgebung führt auch zu einer Zentrierung der Dornfortsätze innerhalb der Stützfläche, so dass dadurch das Einsetzen des Implantates erleichtert wird.

Grundsätzlich ist es möglich, dass jeweils nur ein Stützarm einer Implantatkomponente einer Stützfläche trägt, wobei dann eine Stützfläche an jeder Implantatkomponente vorgesehen wird. Bei einer bevorzugten Ausführungsform ist jedoch vorgesehen, dass jede Implantatkomponente an ihren beiden Stützarmen jeweils eine Stützfläche aufweist, so dass jede Implantatkomponente eine obere und eine untere Stützfläche ausbildet. Auf diese Weise wird an jedem Dornfortsatz die Abstützung jeweils über zwei nebeneinander angeordnete Stützflächen erfolgen, wobei jeweils eine Stützfläche an einer Implantatkomponente und die andere Stützfläche an der anderen Implantatkomponente angeordnet ist.

Es kann vorgesehen werden, dass mindestens ein Stützarm einer Implantätkomponente durch einen an seinem freien Ende beginnenden Längsschlitz in zwei nebeneinander liegende Stützarmabschnitte unterteilt ist. Man könnte auch sagen, dass jeder Stützarm durch ein Paar von nebeneinander liegenden Stützarmen oder sogar durch eine größere Anzahl von nebeneinander liegenden einzelnen Stützarmen gebildet wird.

Dabei ist es vorteilhaft, wenn Stützarme oder Stützarmabschnitte einer Implantatkomponente im Längsschlitz zwischen zwei Stützarmabschnitten der anderen Implantatkomponente angeordnet sind. Dabei greifen also die Stützarme oder Stützarmabschnitte der beiden Implantatkomponenten kammartig ineinander.

Bei einer ersten bevorzugten Ausführungsform ist vorgesehen, dass die Implantatkomponenten im Wesentlichen U-förmig ausgebildet sind mit nebeneinander angeordneten Stützarmen, die beim Annähern der Implantatkomponenten mit ihren Innenseiten an den Aufgleitflächen der anderen Implantatkomponente aufgleiten und dadurch auseinander geschwenkt werden.

Dabei ist es günstig, wenn mindestens ein Stützarm an seiner Außenseite eine Stützfläche trägt.

Bei einer anderen bevorzugten Ausführungsform ist vorgesehen, dass die Stützarme einer Implantatkomponente ausgehend von der sie verbindenden Brücke so gegeneinander geneigt verlaufen, dass sie sich überkreuzen, und dass die Stützarme mit ihren Außenseiten an den Aufgleitflächen der anderen Implantatkomponente aufgleiten und dadurch noch stärker gegeneinander geneigt werden, wobei sich in Folge der Überkreuzung der Stützarme die freien Enden der Stützarme voneinander entfernen. Während bei der vorstehend beschriebenen Ausführungsform also die Stützarme auf ihrer ganzen Länge auseinandergeschwenkt werden und dadurch den Abstand der Stützflächen vergrößern, erfolgt die Vergrößerung des Abstandes der Stützflächen bei der zuletzt beschriebenen Ausführungsform durch verstärke Überkreuzung der Stützarme und damit durch eine Vergrößerung des Abstandes der Stützarme im Bereich ihrer freien Enden.

Auch hier kann vorgesehen sein, dass mindestens ein Stützarm an seiner Innenseite eine Stützfläche trägt.

Bei der überkreuzten Ausführungsform ist es günstig, wenn die Außenseite eines Stützarmes einer Implantatkomponente die Aufgleitfläche für einen Stützarm der anderen Implantatkomponente bildet. Damit gleiten also die Stützarme aneinander auf, jeweils die Außenseite der Stützarme bildet somit die Aufgleitfläche aus, während die Innenseite, die beim Aufgleiten nach außen verschwenkt wird, dann die Stützfläche trägt.

Besonders vorteilhaft ist es, wenn beide Implantatkomponenten eines Implantates gleich ausgebildet sind, dadurch kann das Implantat besonders einfach hergestellt werden, Voraussetzung dafür ist, dass die beiden Implantatkomponenten spiegelbildlich ausgebildet sind, so dass jeweils Stützarme einer Implantatkomponente an Aufgleitflächen der anderen Implantatkomponente aufgleiten können.

Insbesondere sind die Implantatkomponenten einstückig ausgebildet.

Bei einer anderen bevorzugten Ausführungsform ist vorgesehen, dass eine Implantatkomponente aus zwei Einzelteilen besteht, die im Bereich der Brücke scharnierartig miteinander verbunden sind.

Insbesondere können beide Einzelteile an ihren aneinander anliegenden Enden jeweils einen Lagerwulst tragen, die nebeneinander verlaufen und gemeinsam von einer Klammer umfasst und dadurch nebeneinander gehalten werden.

Es ist günstig, wenn die Implantatkomponente aus einem elastisch verformbaren Material besteht.

Bei einer speziellen Ausführungsform ist vorgesehen, dass die Implantatkomponente im Bereich der Brücke eine Verdickung aufweist.

Bei einer weiteren Ausführungsform kann vorgesehen werden, dass zwischen die beiden Stützarme und die Brücke ein den Innenraum der Implantatkomponente ausfüllender Kern eingelegt ist.

Bei einer bevorzugten Ausführungsform weist die Implantatkomponente im Bereich der Brücke mindestens eine Durchbrechung für einen die beiden Implantatkomponenten gegeneinander schiebenden Zuganker auf. Mittels dieses Zugankers kann der Abstand der Implantatkomponenten verringert werden, und dies führt dann zu einer Erhöhung des Abstandes der Stützflächen.

Günstig ist es dabei, wenn durch die Implantatkomponenten mindestens ein Zuganker hindurchgreift, der die beiden Implantatkomponenten gegeneinander spannt.

Bei einer anderen bevorzugten Ausführungsform ist vorgesehen, dass seitlich neben den Implantatkomponenten einseitig oder beidseitig ein Zuganker angeordnet ist, der außen an den Brücken der beiden Implantatkomponenten anliegende Endstücke und dadurch die beiden Implantatkomponenten gegeneinander spannt.

Der Zuganker kann bei einer speziellen Ausführungsform außenseitig von einer Abdeckung überfangen sein, dadurch wird der Zuganker gegenüber seiner Umgebung abgedeckt, so dass ein unerwünschter Kontakt mit umgebendem Gewebe vermieden werden kann.

Die Abdeckung kann beispielsweise leistenförmig ausgebildet sein.

Es ist günstig, wenn der Zuganker sich abwechselnde Vorsprünge und Rücksprünge aufweist und wenn an dem Implantat ein zwischen benachbarte Vorsprünge einrückbares Verriegelungselement gelagert ist. Auf diese Weise kann man nach dem Zusammenschieben der Implantatkomponenten durch Einrücken des Verriegelungselementes die beiden Implantatkomponenten in der einander angenäherten und gegeneinander gespannten Stellung festlegen. Diese Festlegung erfolgt sehr einfach dadurch, dass das Verriegelungselement aus einer nicht eingerückten Stellung in eine eingerückte Stellung verlagert wird.

Bei einer besonders bevorzugten Ausführungsform ist vorgesehen, dass das Verriegelungselement eine Durchbrechung für den Zuganker aufweist mit zwei nebeneinander liegenden Bereichen, von denen ein erster Bereich so groß ist, dass der Zuganker mit seinen Vorsprüngen in axialer Richtung frei durch diesen ersten Bereich hindurchschiebbar ist, während der zweite Bereich nur so groß ist, dass ein zwischen benachbarten Vorsprüngen liegender Abschnitt des Zugankers in ihn einführbar ist, ein einen Vorsprung tragender Abschnitt dagegen nicht. Eine Verschiebung des Verriegelungselementes gegenüber dem Zuganker reicht also aus, um das Verriegelungselement aus einer Freigabestellung, in der der Zuganker durch die Durchbrechung verschiebbar ist, in eine Verriegelungsstellung zu bringen, in der der Zuganker relativ zu dem Verriegelungselement axial unverschieblich festgelegt ist.

Die Vorsprünge können insbesondere als Umfangsrippen des Zugankers ausgebildet sein.

Günstig ist es, wenn das Verriegelungselement um eine parallel zur Längsachse des Zugankers verlaufende Achse verschwenkbar ist. Allein durch eine solche Verschwenkung kann damit das Verriegelungselement von der Freigabestellung in die Verriegelungsstellung verschwenkt werden und umgekehrt.

Bei einer bevorzugten Ausführungsform ist vorgesehen, dass das Verriegelungselement in einer Stellung festlegbar ist, in der es zwischen benachbarte Vorsprünge des Zugankers eingreift, also in seiner Verriegelungsstellung.

Beispielsweise kann zur Festlegung des Verriegelungselementes eine Kappe vorgesehen sein, die das Verriegelungselement und ein daneben angeordnetes, am Zuganker gehaltenes Halteglied umgreifend auf das Verriegelungselement und das Halteglied aufsetzbar ist. Insbesondere kann das Aufsetzen durch ein elastisches Aufschnappen erfolgen, so dass dadurch in einfacher Weise Verriegelungselement und Halteglied relativ zueinander unverschiebbar festgelegt sind.

Es kann vorgesehen sein, dass das Halteglied und das Verriegelungselement relativ zueinander verschwenkbar aneinander gelagert sind, beispielsweise durch einen Lagerzapfen an einem der beiden Teile, der in eine Lagerausnehmung des anderen Teils eingreift.

Bei einer anderen Ausführungsform ist vorgesehen, dass das Implantat in einem dieses umgebenden Gehäuse angeordnet ist, welches einseitig von dem Implantat abziehbar ist. Dadurch wird das Einsetzen des Implantates erleichtert, Teile des Implantates, die gegebenenfalls elastisch von diesem abstehen, können dadurch zunächst in der zusammengelegten Form gehalten werden, so dass die Baugröße des Implantates klein ist und außerdem ein Kontakt mit umgebenden Gewebeteilen vermieden wird. Erst nach dem Einsetzen wird das Gehäuse abgezogen, so dass dann ein Ausschwenken oder Ausfahren von Teilen des Implantates möglich ist.

Gemäß einer besonders bevorzugten Ausführungsform ist vorgesehen, dass an dem Implantat neben den Stützflächen seitliche Anlageelemente für den auf der Stützfläche ruhenden Dornfortsatz angeordnet sind. Diese Anlageelemente legen sich seitlich an den auf der Stützfläche ruhenden Dornfortsatz an und fixieren dadurch das Implantat relativ zum Dornfortsatz.

Dabei ist es vorteilhaft, wenn die Anlageelemente jeweils Teil der Implantatkomponenten sind.

Es kann aber bei anderen Ausführungsformen auch vorgesehen sein, dass die Anlageelemente separate Teile sind, die jeweils an einer Implantatkomponente gehalten sind.

Besonders günstig ist es, wenn die Anlageelemente durch die Relativbewegung der beiden Implantatkomponenten aus einer Anfangsstellung, in der sie wenig oder gar nicht von der Implantatkomponente abstehen, in eine Endstellung bewegbar oder verformbar sind, in der sie stärker von der Implantatkomponente abstehen. Auf diese Weise wird beim Zusammenschieben der beiden Implantatkomponenten nicht nur der Abstand der Stützflächen vergrößert, sondern es werden gleichzeitig auch die Anlageelemente aus der Anfangsstellung in die Endstellung gebracht, in der sie sich seitlich am Dornfortsatz abstützen. Beim Implantieren dagegen stehen die Anlageelemente nicht oder nur geringfügig nach außen über die Kontur des Implantates vor und behindern daher das Einsetzen nicht.

Bei einer ersten bevorzugten Ausführungsform ist vorgesehen, dass ein Anlageelement durch einen Stützarm oder einen Stützarmabschnitt der Implantatkomponente gebildet wird, der keine Stützfläche trägt. Es gibt also bei diesen Ausführungsformen verschiedenartige Stützarme, nämlich einmal Stützarme, die eine Stützfläche ausbilden oder tragen, zum anderen Stützarme, die ein Anlageelement ausbilden.

Dabei ist es günstig, wenn der ein Anlageelement bildende Stützarm oder Stützarmabschnitt flexibeler ausgebildet ist als der die Stützfläche tragende Stützarmabschnitt.

Bei einer anderen bevorzugten Ausführungsform ist vorgesehen, dass jede Implantatkomponente von einer U-förmigen Klammer umgeben ist, die an der Außenseite der Implantatkomponente anliegt und jeweils zur anderen Implantatkomponente weisende, die Anlageelemente bildende Stege trägt, die an Aufgleitflächen der anderen Implantatkomponente oder der an dieser gehaltenen Klammer anliegen und dadurch beim Annähern der Implantatkomponenten von der Anfangsstellung und die Endstellung gelangen. Im Grunde ist dies eine ähnliche Ausgestaltung wie bei den Implantatkomponenten, bei denen die Stützarme beim Annähern der Implantatkomponenten verschoben werden, bei dieser Ausgestaltung erfolgt ein ähnliches Verschieben auch bei den Stegen, die die U-förmige Klammer trägt, die die Implantatkomponente außenseitig umgibt.

Bei einer weiteren bevorzugten Ausführungsform ist vorgesehen, dass jeweils an die Außenseite der Brücke der beiden Implantatkomponenten Spannelemente angelegt sind, die bei Annäherung die beiden zwischen den Spannelementen angeordneten Implantatkomponenten gegeneinander spannen, und dass die Spannelemente mit die Anlageelemente bildenden Stegen an der Außenseite der Brücke anliegen, die bei Annäherung an der Außenseite der Brücken aufgleiten und dadurch von der Anfangsstellung in die Endstellung gelangen. In diesem Falle bilden also die Brücken der Implantatkomponenten die Aufgleitflächen für die Stege der Spannelemente, die dadurch beim Zusammenschieben nach außen gebogen werden und die Anlageelemente bilden.

Ferner ist offenbart, dass die Anlageelemente zwischen den Stützarmen einer Implantatkomponente und der Aufgleitfläche der anderen Implantatkomponente im Klemmsitz gehalten sind. In dieser Position werden die Anlageelemente eingeschoben und werden dann durch das Zusammenspannen der Implantatkomponenten in dieser Lage fixiert.

Günstig ist es dabei, wenn die Anlageelemente an gegenüberliegenden Enden nach entgegengesetzten Seiten abgebogen sind, so dass die beiden Enden dann auf gegenüberliegenden Seiten des Implantats entsprechende Anlageelemente ausbilden.

Insbesondere können die Anlageelemente bandförmig ausgebildet sein.

Es ist günstig, wenn mindestens ein Anlageelement einen Längsschlitz aufweist. Es ergeben sich dann zwei parallel zueinander verlaufende, stegförmige Abschnitte an dem Anlageelement.

Insbesondere kann dabei vorgesehen sein, dass ein Teil einer Implantatkomponente in den Längsschlitz hineinragt und an den Seitenkanten des Längsschiitzes anliegt. Dadurch wird das Anlageelemente relativ zu der Implantatkomponente bei einer Längsverschiebung geführt, da der in den Längsschlitz hineinragende Teil der Implantatkomponente als Führungselement wirkt.

Bei einer besonderen Ausgestaltung ist vorgesehen, dass die Anlageelemente eine einseitige Verdickung tragen, durch die ein Herausziehen des Anlageelementes zwischen dem Stützarm einer Implantatkomponente und der Anlagefläche der anderen Implantatkomponente in die Endstellung begrenzt wird.

Ferner ist offenbart, dass die Anlageelemente in der Anfangsstellung flächig an den Stützarmen anliegen. Dadurch ergibt sich eine besonders geringe Bauhöhe des gesamten Implantates.

Bei einer weiteren bevorzugten Ausführungsform ist vorgesehen, dass in den Innenraum einer Implantatkomponente zwischen der Brücke und dem brückenseitigen Teil der Stützarme eine Schraubenfeder eingesetzt ist und mit ihren Enden durch Durchbrechungen der Stützarme aus dem Innenraum herausragt, die die Anlageelemente bilden. Diese federnden Enden können beim Implantieren umgebogen sein, so dass sie das Einführen nicht behindern, nach dem Einsetzen des Implantates werden diese freien Enden freigegeben und schwenken aus und bilden dann die an dem Dornfortsatz anliegenden Anlageelemente.

Bei einer weiteren bevorzugten Ausführungsform ist das Anlageelement in einer Führung der Implantatkomponente verschiebbar gelagert, ragt mit einem Ende aus der Implantatkomponente heraus und ist mit dem andern Ende an einem Endstück gehalten, welches an der Außenseite der Implantatkomponente aniiegt. Außerdem ist ein Zugmittel vorgesehen, welches die Endstücke einander annähert, die Implantatkomponenten dadurch gegeneinander spannt und gleichzeitig das Anlageelement in der Führung aus der Anfangsstellung in die Endstellung verschiebt. Auch hier wird also die Relativverschiebung der beiden Implantatkomponenten ausgenützt, um gleichzeitig das Anlageelement von der Anfangsstellung in die Endstellung zu verschieben.

Die Führungen können dabei durch Durchbrechungen in der Brücke und in einem Stützarm gebildet werden, durch die das Anlageelement hindurchragt.

Die nachfolgende Beschreibung bevorzugter Ausführungsformen der Erfindung dient im Zusammenhang mit der Zeichnung der näheren Erläuterung, wobei die Figuren 1-9 und 48-53 jedoch nicht die Erfindung darstellen. Es zeigen :
- Figur 1:: eine perspektivische Ansicht von zwei benachbarten Wirbelkörpern mit einem zwischen die Dornfortsätze der Wirbelkörper eingesetzten Implantat vor dem Zusammenschieben der beiden Implantatkomponenten und vor dem Aufrichten der seitlichen Anlageelemente;
- Figur 2:: eine Ansicht ähnlich Figur 1 nach dem Zusammenschieben der beiden Implantatkomponenten und nach dem Aufrichten der seitlichen Anlageelemente;
- Figur 3:: eine perspektivische Ansicht des Implantates der Figuren 1 und 2 vor dem Zusammenschieben der Implantatkomponenten und vor dem Einsetzen der seitlichen Anlageelemente;
- Figur 4:: eine Ansicht ähnlich Figur 3 bei teiweise zusammengeschobenen Implantatkomponenten und mit eingesetzten, aber noch nicht vollständig aufgerichteten Anlageelementen;
- Figur 5:: eine Ansicht ähnlich Figur 4 mit vollständig zusammengeschobenen Implantatkomponenten und aufgerichteten Anlageelementen;
- Figur 6:: eine Explosionsdarstellung des Implantates der Figur 3;
- Figur 7:: eine Ansicht ähnlich Figur 3 mit einem weiteren bevorzugten Ausführungsbeispiel eines Implantates;
- Figur 8:: eine Ansicht ähnlich Figur 7 bei zusammengeschobenen Implantatkomponenten und aufgerichteten Anlageelementen;
- Figur 9:: eine Explosionsdarstellung des Implantates der Figuren 7 und 8;
- Figur 10:: eine perspektivische Ansicht eines Implantates mit zwei Implantatkomponenten bei Beginn der Annäherung der beiden Implantatkomponenten;
- Figur 11:: eine Ansicht der beiden Implantatkomponenten des Implantates der Figur 10 vor deren Zusammenschieben;
- Figur 12:: eine perspektivische Ansicht ähnlich Figur 10 bei einem weiteren bevorzugten Ausführungsbeispiel eines Implantates;
- Figur 13.: eine perspektivische Ansicht der Implantatkomponenten der Figur 12 vor dem Zusammenschieben;
- Figur 14:: eine Ansicht ähnlich Figur 10 bei einem weiteren bevorzugten Ausführungsbeispiel eines Implantates;
- Figur 15:: eine perspektivische Ansicht der Implantatkomponenten der Figur 14 vor dem Zusammenschieben;
- Figur 16:: eine perspektivische Ansicht eines weiteren bevorzugten Ausführungsbeispieles eines Implantates mit einem die Implantatkomponenten durchsetzenden Zuganker;
- Figur 17:: eine Explosionsansicht des Implantates der Figur 16;
- Figur 18:: eine perspektivische Ansicht eines weiteren bevorzugten Ausführungsbeispieles eines Implantates mit zusammengeschobenen Implantatkomponenten mit jeweils paarweise ausgebildeten Stützarmen;
- Figur 19:: eine perspektivisch Ansicht der Implantatkomponenten des Implantates der Figur 18 vor dem Zusammenschieben;
- Figur 20:: eine perspektivische Ansicht ähnlich Figur 18 bei einem weiteren bevorzugten Ausführungsbeispiel eines Implantates mit unterschiedlich ausgebildeten Stützarmen;
- Figur 21:: eine perspektivische Ansicht der Implantatkomponenten des Implantates in Figur 20 vor dem Zusammenschieben;
- Figur 22:: eine perspektivische Ansicht eines weiteren bevorzugten Ausführungsbeispieles eines Implantates mit zusammengeschobenen Implantatkomponenten und mit eingesetzten und aufgerichteten Anlageelementen in Form von Schraubenfedern;
- Figur 23:: eine Explosionsansicht des Implantates der Figur 23;
- Figur 24:: eine perspektivische Ansicht eines weiteren bevorzugten Ausführungsbeispiels eines Implantates in einer Einsetzhülse;
- Figur 25:: eine perspektivische Ansicht des Implantates der Figur 24 nach dem Herausziehen aus der Einsetzhülse;
- Figur 26:: eine Explosionsansicht des Implantates der Figuren 24 und 25;
- Figur 27:: eine perspektivische Ansicht eines weiteren bevorzugten Ausführungsbeispiels eines Implantates mit auf die Implantatkomponenten aufgesetzten Klammern mit armförmigen Anlageelementen;
- Figur 28:: eine Explosionsansicht des Implantates der Figur 27;
- Figur 29:: eine perspektivische Ansicht eines weiteren bevorzugten Ausführungsbeispieles eines Implantates mit klammerförmigen, neben den Implantatkomponenten angeordneten Anlageelementen;
- Figur 30:: eine Explosionsansicht des Implantates der Figur 29;
- Figur 31:: eine perspektivische Ansicht eines weiteren bevorzugten Ausführungsbeispiels eines Implantates mit Stützarmen, die sich nicht überkreuzen;
- Figur 32:: eine Explosionsansicht der Implantatkomponenten des Implantates der Figur 31;
- Figur 33:: eine perspektivische Ansicht ähnlich Figur 31 einer weiteren bevorzugten Ausführungsform eines Implantates;
- Figur 34:: eine Explosionsansicht des Implantates der Figur 33;
- Figur 35:: eine perspektivische Ansicht eines Implantates ähnlich Figur 33 mit in den Innenraum eingesetzten Kernen;
- Figur 36:: eine Explosionsansicht des Implantates der Figur 35;
- Figur 37:: eine perspektivische Ansicht eines weiteren bevorzugten Ausführungsbeispiels eines Implantates in einem Einsetzgehäuse;
- Figur 38:: eine perspektivische Ansicht des Implantates der Figur 37 nach dem Herausziehen aus dem Einsetzgehäuse;
- Figur 39:: eine Explosionsansicht des Implantates der Figur 38;
- Figur 40:: eine perspektivische Ansicht einer weiteren bevorzugten Ausführungsform eines Implantates;
- Figur 41:: eine Explosionsansicht des Implantates der Figur 40;
- Figur 42:: eine perspektivische Ansicht eines weiteren bevorzugten Ausführungsbeispiels eines Implantates mit flächig an den Stützarmen anliegenden Anlageelementen;
- Figur 43:: eine perspektivische Ansicht des Implantates der Figur 42 nach dem Zusammenschieben der Implantatkomponenten und nach dem Aufrichten der Anlageelemente;
- Figur 44:: eine Explosionsdarstellung des Implantates der Figuren 42 und 43;
- Figur 45:: eine perspektivische Ansicht eines weiteren beorzugten Ausführungsbeispieles eines Implantates vor dem Zusammenschieben der Implantatkomponenten;
- Figur 46:: eine perspektivische Ansicht des Implantates der Figur 45 nach dem Zusammenschieben der beiden Implantatkomponenten ;
- Figur 47:: eine Explosionsdarstellung des Implantates der Figuren 45 und 46;
- Figur 48:: eine perspektivische Ansicht eines weiteren bevorzugten Ausführungsbeispiels eines Implantates nach dem Zusammenschieben der Implantatkomponenten;
- Figur 49:: eine Explosionsdarstellung des Implantates der Figur 48;
- Figur 50:: eine perspektivische Ansicht eines weiteren bevorzugten Ausführungsbeispiels eines Implantates nach dem Zusammenschieben der Implantatkomponenten;
- Figur 51:: eine Explosionsdarstellung des Implantates der Figur 50;
- Figur 52:: eine perspektivische Ansicht der Zuganker und der Verriegelungseinrichtung für die Zuganker bei den Ausführungsbeispielen der Figuren 48 bis 51 vor dem Verriegeln der Zuganker und
- Figur 53:: eine Ansicht ähnlich Figur 52 nach dem Verriegeln der Zuganker und vor dem Sichern des Verriegelungselementes.

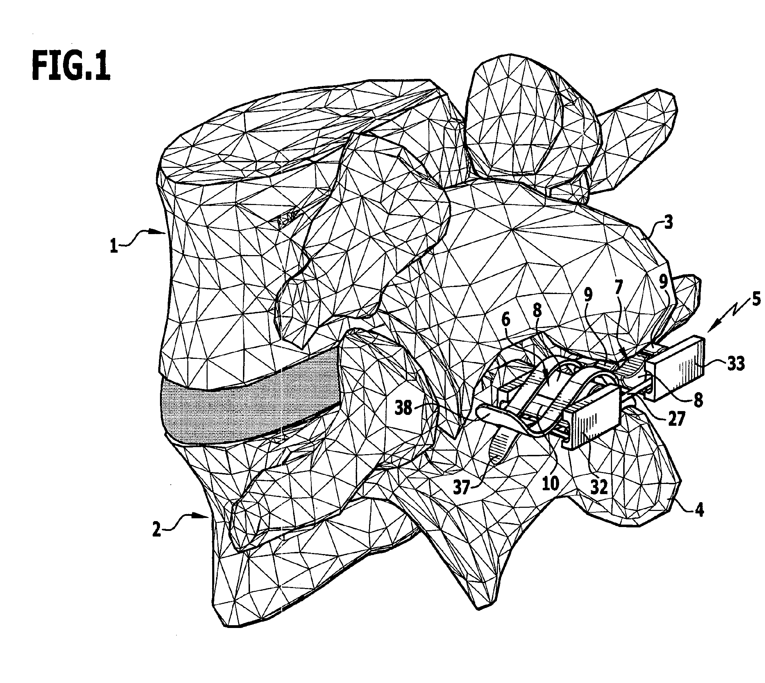

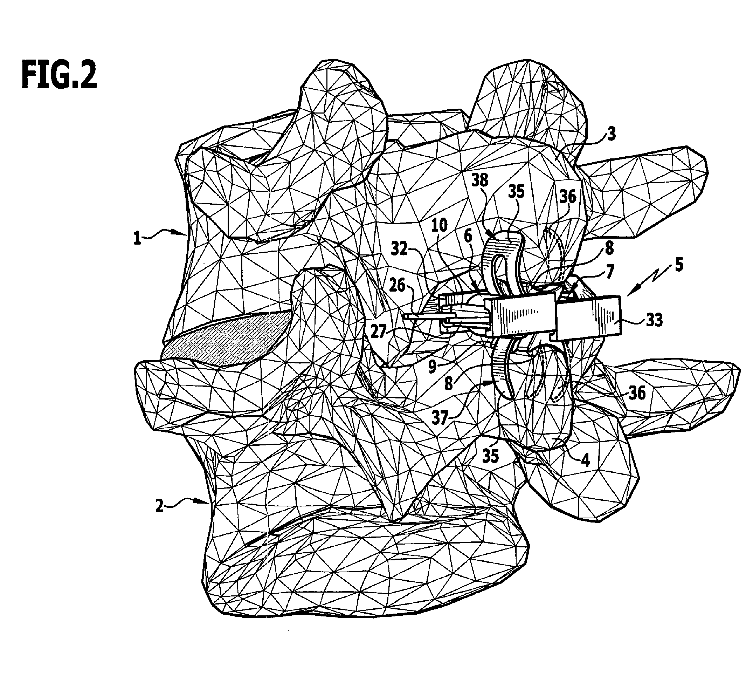

In den Figuren 1 und 2 sind perspektivisch zwei nebeneinander liegende Wirbelkörper 1, 2 dargestellt, zwischen deren Dornfortsätze 3, 4 ein Implantat 5 eingesetzt ist. Dieses stützt sich an beiden benachbarten Dornfortsätzen 3, 4 ab und wirkt dort als Distanzelement zwischen den beiden Dornfortsätzen 3, 4, so dass durch Veränderung der Höhe des Implantates 5 der Abstand der beiden Dornfortsätze 3, 4 eingestellt und dann durch das Implantat 5 aufrechterhalten werden kann.

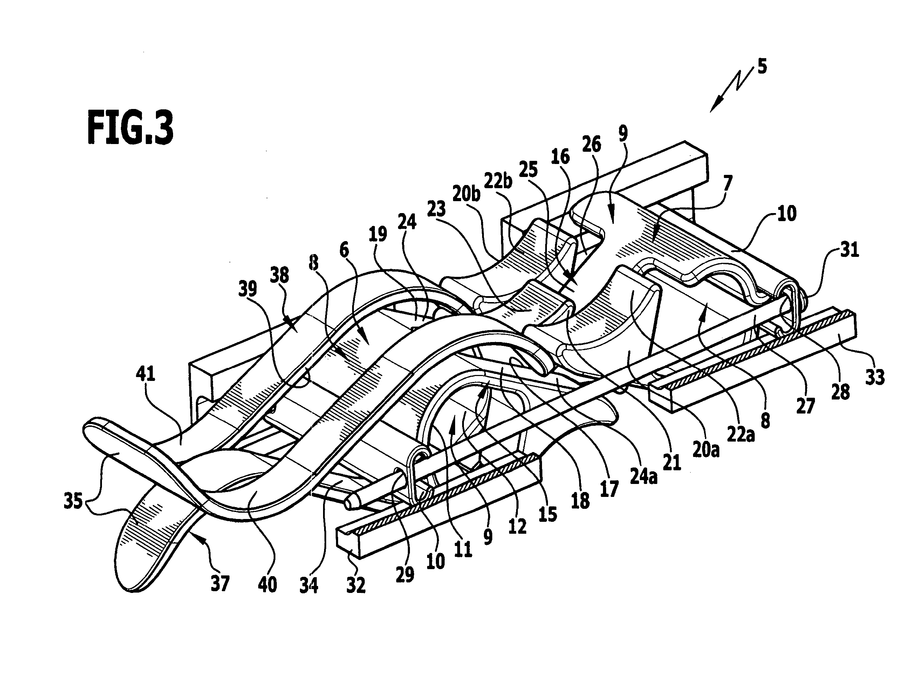

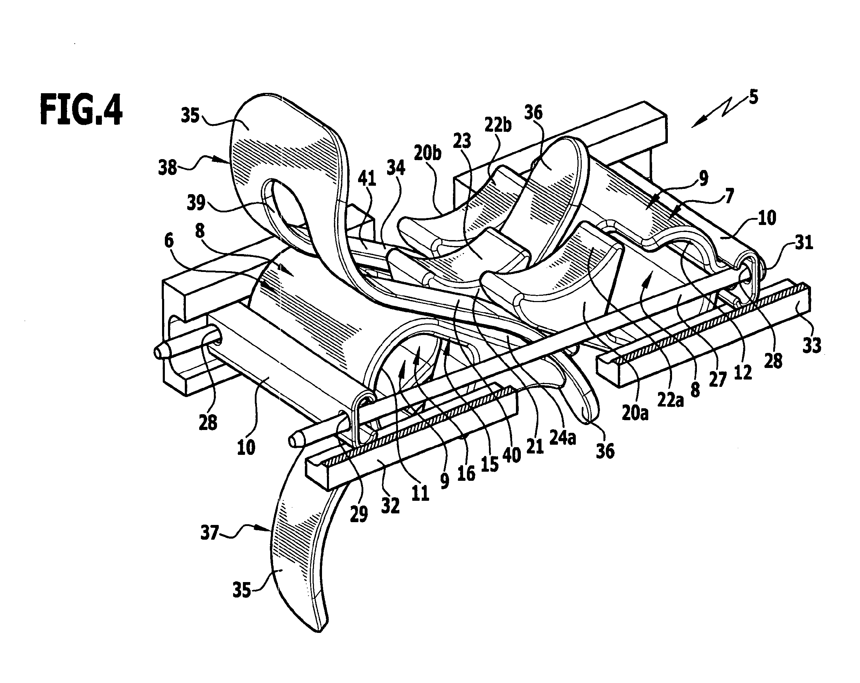

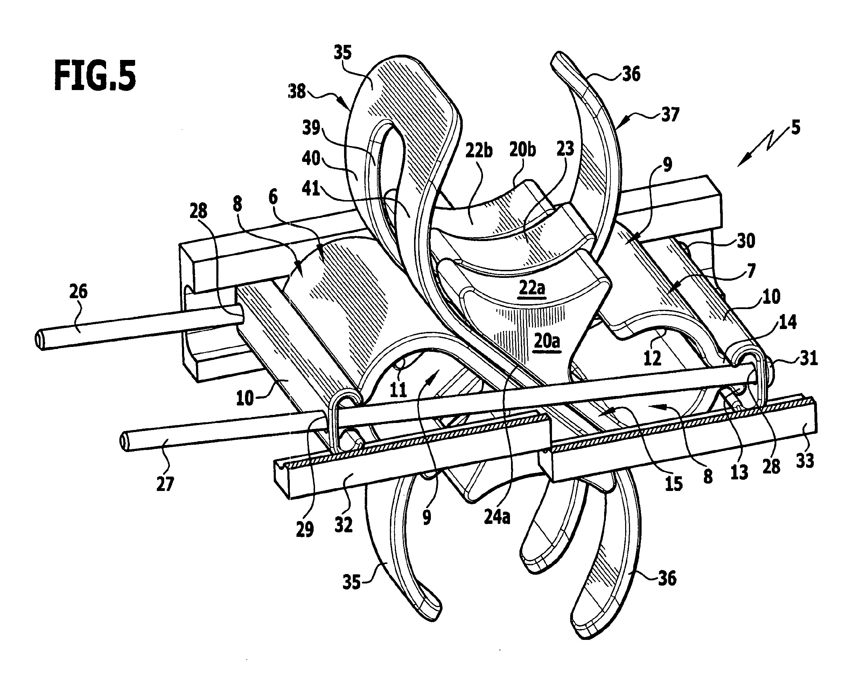

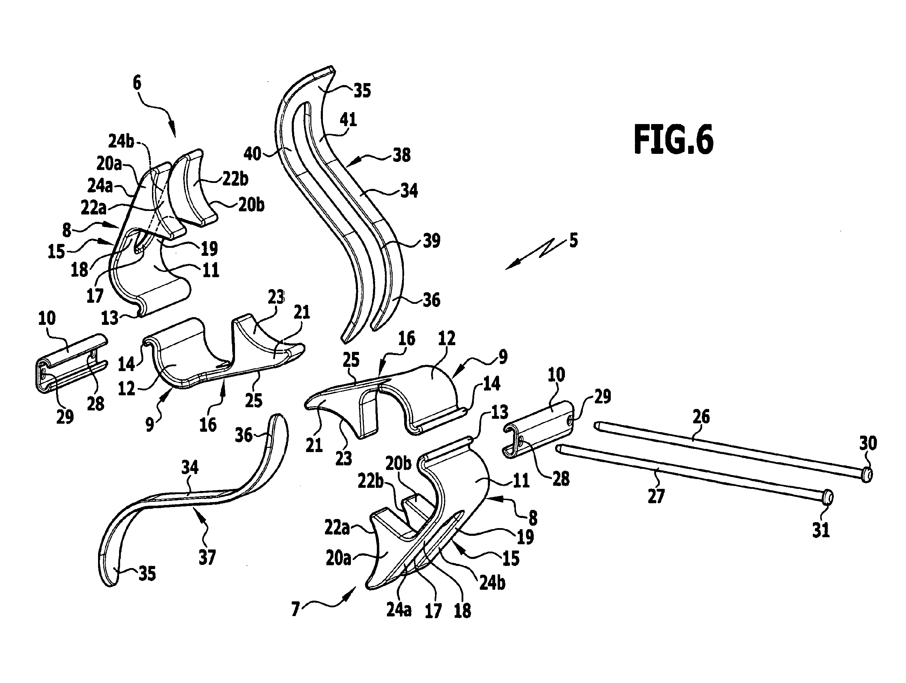

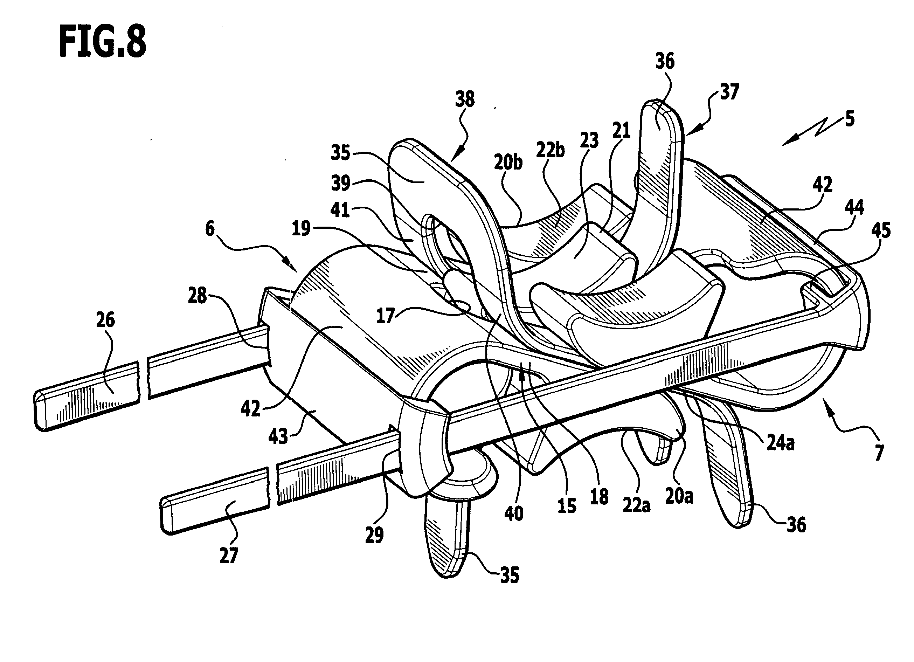

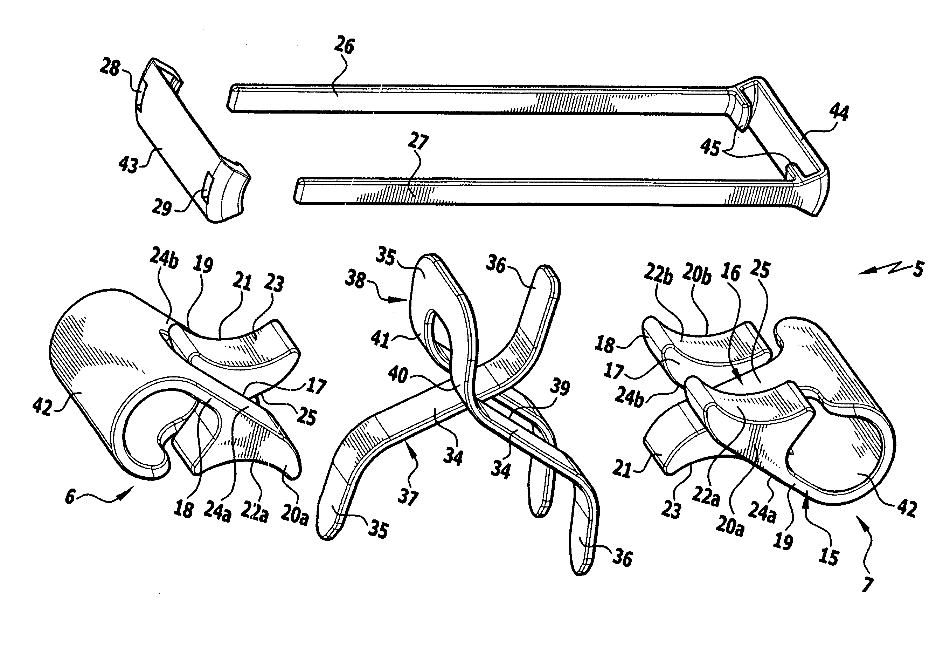

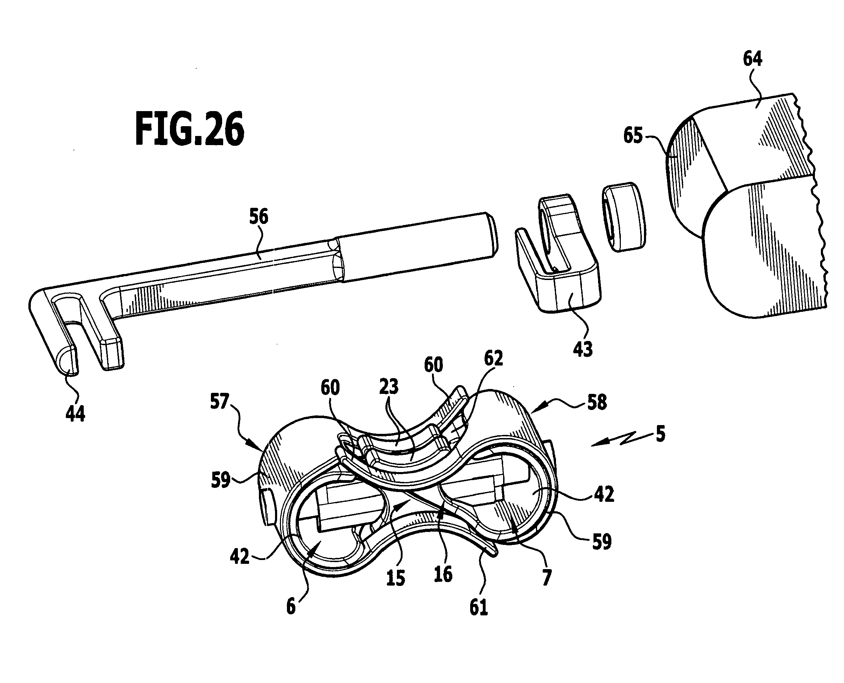

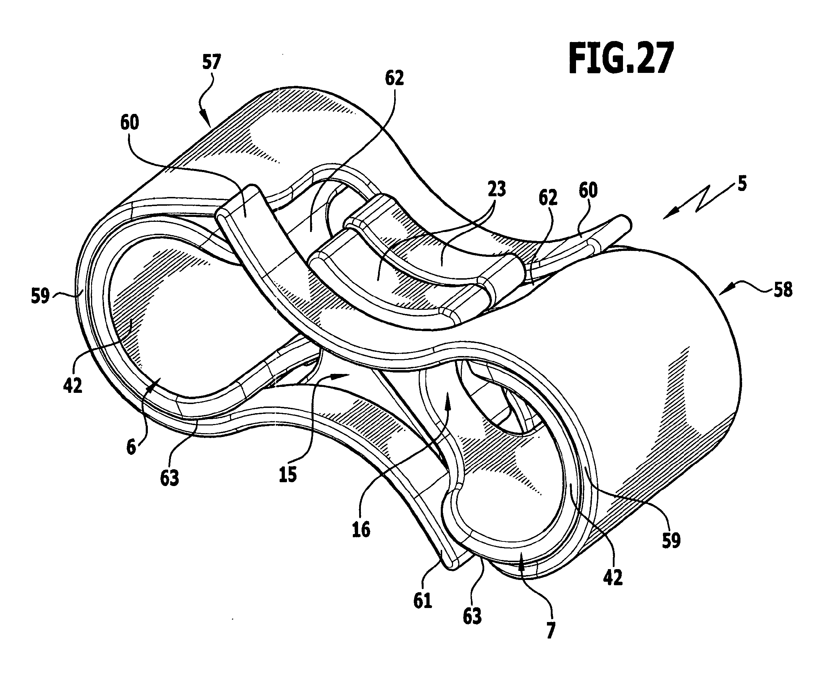

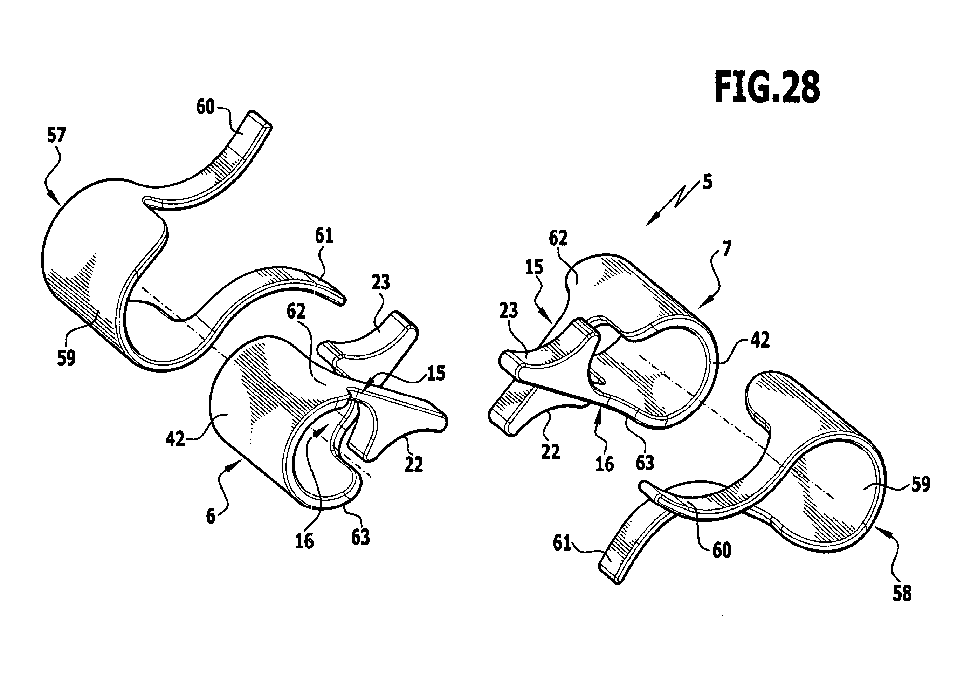

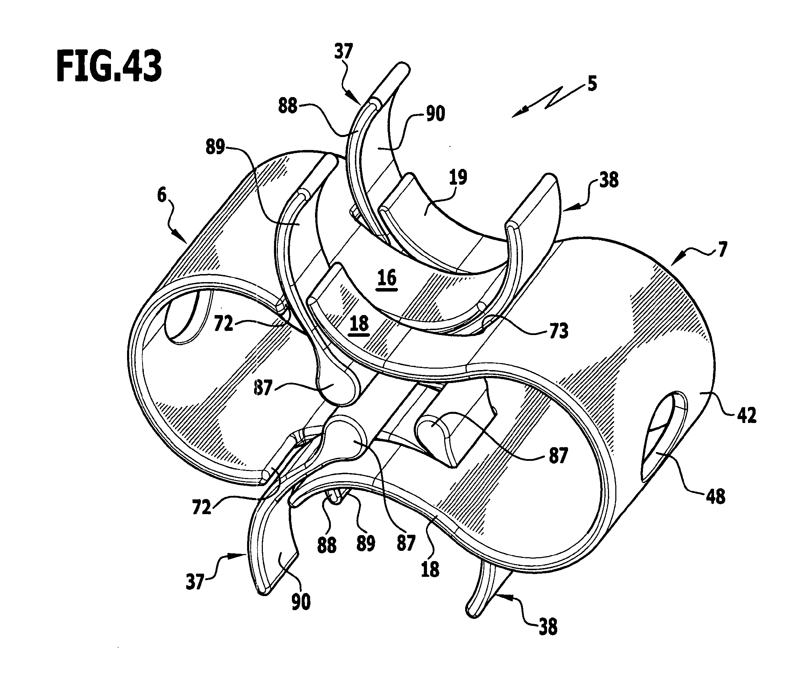

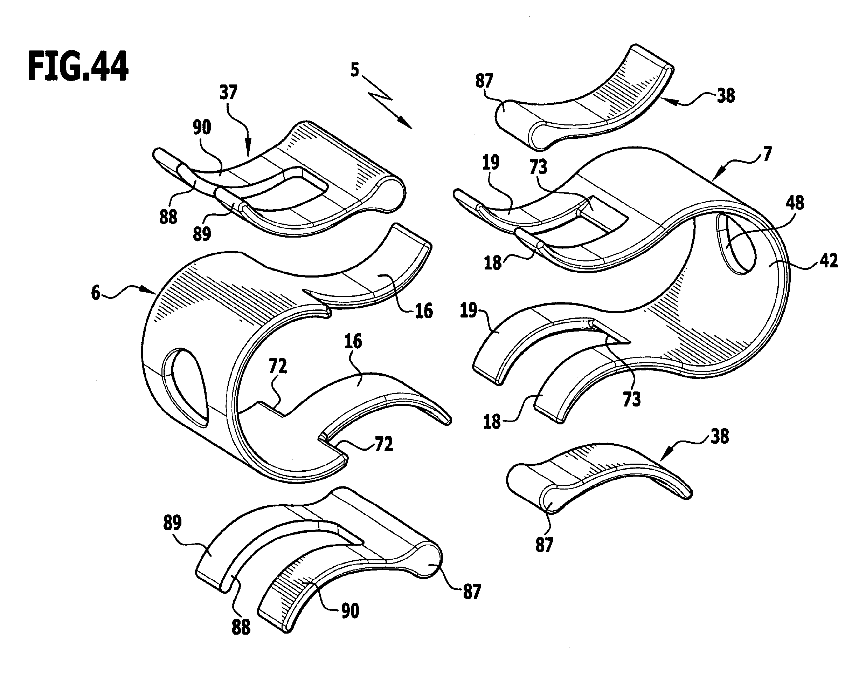

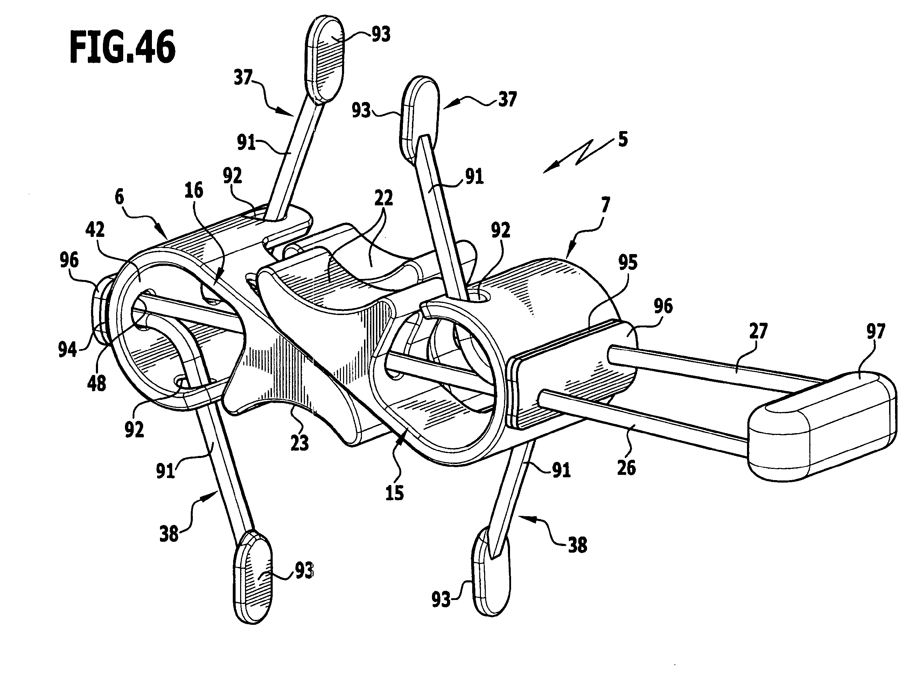

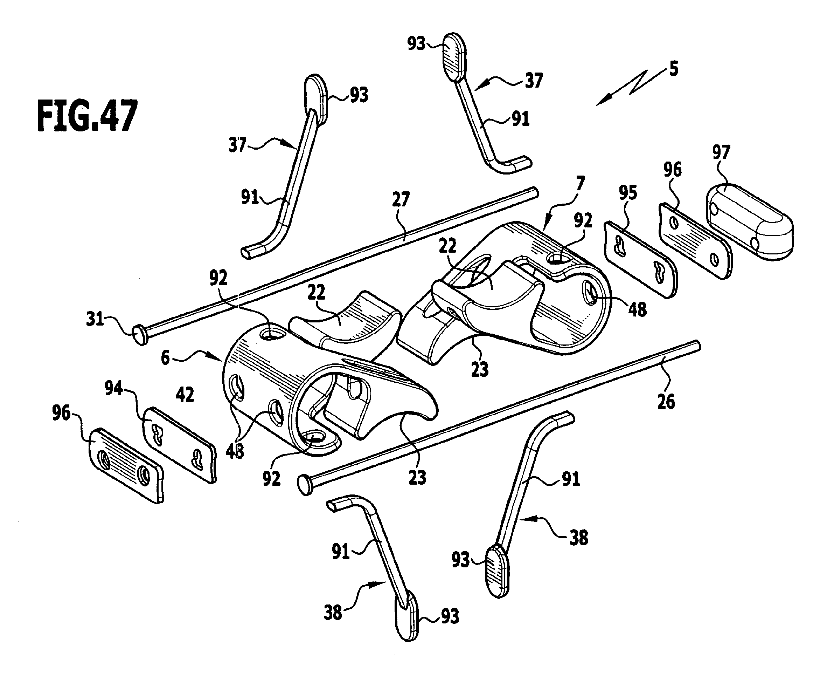

Der Aufbau dieses Implantates 5 kann verschieden gewählt werden, nachstehend werden an Hand der Figuren 3 bis 47 unterschiedliche Ausführungsformen eines derartigen Implantates 5 beschrieben. Allen Implantaten gemeinsam ist die Fähigkeit, dass das Implantat bei geringer Bauhöhe in den Zwischenraum zwischen zwei Dornfortsätzen 3, 4 eingesetzt werden kann und dann nach dem Einsetzen seine Bauhöhe so vergrößern kann, dass der gewünschte Abstand der Dornfortsätze 3, 4 eingestellt und aufrechterhalten werden kann.

Bei einem ersten bevorzugten Ausführungsbeispiel, das in den Figuren 1 bis 6 dargestellt ist, umfasst das Implantat 5 zwei in diesem Falle gleich aufgebaute Implantatkomponenten 6, 7, von denen nachstehend nur eine näher beschrieben wird. Diese Implantatkomponente ist aus zwei getrennten Bauteilen 8, 9 zusammengesetzt, die über eine C-förmige Klammer 10 zusammengehalten werden. Beide Bauteile 8, 9 weisen an den einander zugewandten Enden der Bauteile 8, 9 einen bandförmigen, abgebogenen Abschnitt 11 beziehungsweise 12 auf, der am Ende der Bauteile 8, 9 in einem in entgegengesetzter Richtung stark abgebogenen Flansch 13, 14 endet. Dieser Flansch kann zusätzlich wulstförmig verdickt sein. Die beiden Flansche 13, 14 werden von der C-förmigen Klammer 10 umgriffen und hintergriffen, so dass in diesem Bereich eine Verbindung der beiden Bauteile 8, 9 erfolgt, die auch ein Verschwenken der Bauteile 8, 9 gegeneinander ermöglicht, es handelt sich also um eine scharnierähnliche Verbindung.

Beide gebogenen Abschnitte 11, 12 gehen an ihrem den Flanschen 13 beziehungsweise 14 gegenüberliegenden Ende in Stützarme 15, 16 über, wobei an einem Abschnitt 11 der Stützarm 15 sich über die gesamte Breite des Abschnittes 11 erstreckt und durch einen vom freien Ende des Bauteiles 8 bis zum Beginn des Abschnittes 11 sich erstreckenden Längsschlitz 17 in zwei nebeneinander liegende Stützarmabschnitte 18, 19 unterteilt ist. Bei dem anderen Bauteil 9 ist der Stützarm 16 schmaler ausgebildet als der Abschnitt 12 und in dessen Mitte angeordnet, die Breite des Stützarmes 16 ist dabei gleich oder kleiner als die Breite des Längsschlitzes 17 an dem anderen Bauteil, so dass der Stützarm 16 in den Längsschlitz 17 zwischen die beiden Stützarmabschnitte 18, 19 eintauchen kann.

Sowohl der Stützarm 16 als auch die beiden Stützarmabschnitte 18, 19 tragen an ihren einander zugewandten Innenseiten im Bereich ihrer freien Enden einen Vorsprung 20a, 20b beziehungsweise 21, dessen eine Seite als konkav gebogene Stützfläche 22a, 22b beziehungsweise 23 ausgebildet ist.

Wenn die beiden Bauteile 8, 9 durch die Klammer 10 zusammengehalten sind, überkreuzen sich die Stützarme 15 und 16. Dabei taucht der Stützarm 16 in den Längsschlitz 17 zwischen den beiden Stützarmabschnitten 18, 19 des anderen Bauteils ein. Die Stützflächen 22a, 22b und 23 weisen dadurch jeweils auf der gegenüberliegenden Seite des Bauteiles nach außen. Die beiden gleich aufgebauten Implantatkomponenten 6, 7 werden mit den freien Enden ihrer Stützarme 15, 16 aufeinander zugerichtet so gegeneinander geschoben, dass jeweils die Außenflächen der Stützarme der einen Implantatkomponente an den Außenflächen der Stützarme der jeweils anderen Implantatkomponente anliegen und bei Annäherung der beiden Implantatkomponenten 6, 7 aneinander aufgleiten. Bei dem in den Figuren 1 bis 6 dargestellten Ausführungsbeispiel sind diese Außenflächen im Wesentlichen eben ausgebildet und verlaufen daher aufgrund der Überkreuzung der Stützarme 15, 16 schräg, die Außenflächen der Stützarme 15, 16 einer Implantatkomponente sind dabei zum freien Ende hin etwa V-förmig angeordnet. An den freien Enden sind die Außenflächen 24a, 24b und 25 geringfügig abgebogen, um beim ersten Annähern der Implantatkomponenten 6, 7 das Aufgleiten zu erleichtern.

Beim Zusammenschieben der beiden Implantatkomponenten 6, 7 und beim Aufgleiten der Stützarme 15, 16 aufeinander sind die Stützarme 15, 16 stärker überkreuzt, so dass sich die freien Enden der Stützarme 15, 16 und damit die nach außen weisenden Stützflächen 22a und 22b einerseits sowie 23 andererseits voneinander entfernen, das heißt der Abstand der nach außen weisenden, nach gegenüberliegenden Seiten der Implantatkomponente weisenden Stützflächen wird vergrößert. Dabei liegen die Stützflächen 22a und 22b einer Implantatkomponente und die Stützfläche 23 der anderen Implantatkomponente dann unmittelbar nebeneinander, die Stützfläche 23 der anderen Implantatkomponente befindet sich dabei zwischen den Stützflächen 22a und 22b der einen Implantatkomponente, so dass die drei Stützflächen gemeinsam einemuldenförmige Lagerfläche für einen Dornfortsatz ausbilden, der sich auf diesen nebeneinander liegenden Stützflächen abstützt.

Je weiter die beiden Implantatkomponenten 6, 7 gegeneinander geschoben werden, desto größer kann der Abstand der Stützflächen auf gegenüberliegenden Seiten der Implantatkomponente eingestellt werden und damit desto größer die Bauhöhe des Implantates und der Abstand der sich auf dem Implantat abstützenden Dornfortsätze.

Um die Implantatkomponenten 6, 7 in der beschriebenen Weise gegeneinander zu schieben, sind auf beiden Seiten neben den Implantatkomponenten Zuganker 26, 27 in Form von dünnen Stäben angeordnet, die Durchbrechungen 28, 29 durchsetzen, die sich an den Enden der beiden Klammern 10 befinden. Diese Klammern so halten also einerseits die beiden Bauteile einer Implantatkomponente zusammen, andererseits wirken sie als Endstücke, durch deren Annäherung die beiden Implantatkomponenten 6, 7 gegeneinander geschoben werden.

Beide Zuganker 26, 27 haben an einem Ende einen verdickten Kopf 30 beziehungsweise 31, der die Eintauchtiefe in die Durchbrechungen der einen Klammer begrenzt, auf dem gegenüberliegenden Ende ist die gegenüberliegende Klammer des Implantates frei verschiebbar und kann dort mittels eines in der Zeichnung nicht dargestellten Instrumentes in Richtung auf die andere Klammer verschoben werden, so dass dadurch die beiden Implantatkomponenten gegeneinander geschoben werden. Wenn die gewünschte Einschubtiefe erreicht ist, kann die verschiebbare Klammer durch geeignete Mittel in ihrer Lage fixiert werden, diese Mittel sind in der Zeichnung nicht dargestellt. Es kann sich dabei beispielsweise um eine Verklemmung, eine Verformung oder ähnliches handeln oder der Abstand wird fixiert durch Aufsetzen eines Anschlages auf die Zuganker.

Bei dem in den Figuren 1 bis 6 dargestellten Ausführungsbeispiel werden die Zuganker 26, 27 außenseitig jeweils von einer C-förmigen Leiste 32, 33 abgedeckt, so dass einerseits die Zuganker geschützt sind und andererseits auch ein unerwünschter Kontakt mit umgebendem Gewebe vermieden werden kann.

Bei dem Ausführungsbeispiel der Figuren 1 bis 6 sind zusätzlich bandförmige Anlageelemente in das Implantat eingeschoben, und zwar ein bandförmiges, im mittleren Abschnitt 34 geradliniges und in den beidseitig daran anschließenden Endabschnitten 35, 36 nach gegenüberliegenden Seiten abgebogenes Anlageelement 37, welches im Wesentlichen gleich ausgebildet ist wie ein zweites Anlageelement 38, welches jedoch breiter ist als das Anlageelement 37 und einen von einem Ende fast bis zum anderen Ende verlaufenden Längsschlitz 39 aufweist, so dass dieses breitere Anlageelement 38 in zwei nebeneinander liegende Stege 40, 41 unterteilt ist. Die Breite des Längsschlitzes 39 ist gleich oder größer als die Breite des schmaleren Anlageelementes 37, so dass dieses in den Längsschlitz 39 eintauchen kann.

Das schmalere Anlageelement 37 wird zwischen die Außenflächen der mittleren Stützarme 16 der beiden Implantatkomponenten eingeschoben, die beiden Stege 40 und 41 zwischen die Außenflächen der Stützarmabschnitte 18, 19 der beiden Implantatkomponenten, so dass die nach entgegengesetzten Richtungen gebogenen Endabschnitte 35, 36 der beiden Anlageelemente 37, 38 unmittelbar neben den Stützflächen 22a, 22b und 23 im Wesentlichen quer zur Verschiebeebene der beiden Implantatkomponenten abstehen und sich dadurch seitlich an die Dornfortsätze 3, 4 anlegen, die bei eingesetztem Implantat auf den Stützflächen 22a, 22b und 23 aufruhen. Dadurch wird das Implantat auch gegen eine Querschiebung relativ zu den Dornfortsätzen zuverlässig gesichert, die beiden Anlageelemente 37, 38 werden dabei zwischen den beiden Implantatkomponenten im Klemmsitz gehalten und dadurch gegen jede weitere Verschiebung fixiert.

Bei der Implantation des Implantates sind die Anlageelemente noch nicht eingesetzt, so dass das Implantat aufgrund seiner geringen Bauhöhe einfach durch eine relativ kleine Zugangsöffnung eingesetzt werden kann. Die Anlageelemente 37, 38 werden erst zwischen die Außenflächen der Stützarme eingeschoben, wenn das Implantat eingesetzt ist, allerdings erfolgt dieses Einsetzen vor dem Zusammenschieben der Implantatkomponenten und damit vor der Vergrößerung des Abstandes der Stützflächen, so dass das Anlageelement zwischen den Außenflächen der Stützarme noch frei verschoben werden kann, wie dies an Hand der Figuren 3, 4 und 5 dargestellt ist.

Das beschriebene Implantat kann aus Metall oder einem sterilisierbaren, körperverträglichen Kunststoffmaterial hergestellt werden, es ergibt sich ein sehr einfacher Aufbau, da beide Implantatkomponenten gleich aufgebaut sind. Das Implantat kann in vormontiertem Zustand eingesetzt werden, also mit durch die beiden Zuganker 26, 27 zusammengehaltenen Implantatkomponenten, die aber noch nicht so weit zusammengeschoben sind, dass die Stützflächen wesentlich voneinander entfernt sind.

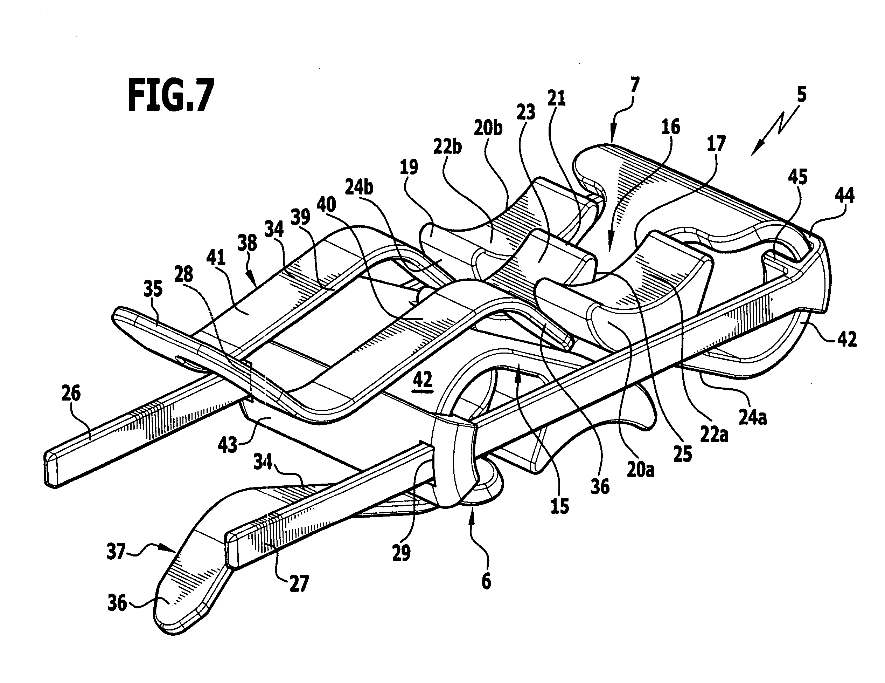

Bei dem Implantat der Figuren 7 bis 9 ist ein sehr ähnlicher Aufbau gewählt, einander entsprechende Teile tragen daher - wie auch bei den nachstehend beschriebenen Ausführungsbeispielen - dieselben Bezugszeichen. Im Unterschied zu dem Ausführungsbeispiel der Figuren 1 bis 6 sind die Implantatkomponenten bei dem Ausführungsbeispiel der Figuren 7 bis 9 und bei allen anderen nachstehend beschriebenen Ausführungsformen nicht aus zwei Bauteilen ausgebildet, sondern einteilig. Die Stützarme 15 und 16 jeder Implantatkomponente sind somit durch einen bandförmigen, einteiligen Brückenabschnitt 42 miteinander verbunden, eine Klammer zum Zusammenhalten von Einzelteilen einer Implantatkomponente ist daher nicht mehr notwendig. Um die beiden Implantatkomponenten zusammenzuschieben, genügt es daher, an der Außenseite der Brückenabschnitte 42 Endstücke 43, 44 vorzusehen, dabei handelt es sich einfach um quer zur Längsrichtung der bandförmigen Brückenabschnitte 42 verlaufende Stege, von denen einer einstückig mit den beiden Zugankern 26, 27 verbunden ist, während der andere Durchbrechungen 28, 29 aufweist, durch die die Zuganker 26, 27 hindurchtreten. Zur Fixierung des die Zuganker 26, 27 tragenden Endstückes 44 am Implantat können parallel zu diesem Endstück 44 verlaufende Vorsprünge 45 an den Zugankern 26, 27 angeformt sein, die an der Innenseite der Brückenabschnitte 42 anliegen, so dass die Seitenkanten der Brückeabschnitte 42 von den Zugankern 26 und 27 und diesen Vorsprüngen 45 umgriffen werden.

Bei den nachstehend beschriebenen Ausführungsform können die beiden Implantatkomponenten in der gleichen Weise gegeneinander verschoben werden, zur Erhöhung der Übersichtlichkeit sind die Endstücke 43, 44 und die Zuganker 26, 27 bei den nachfolgenden Ausführungsbeispielen zum Teil nicht dargestellt und werden dort nicht gesondert beschrieben.

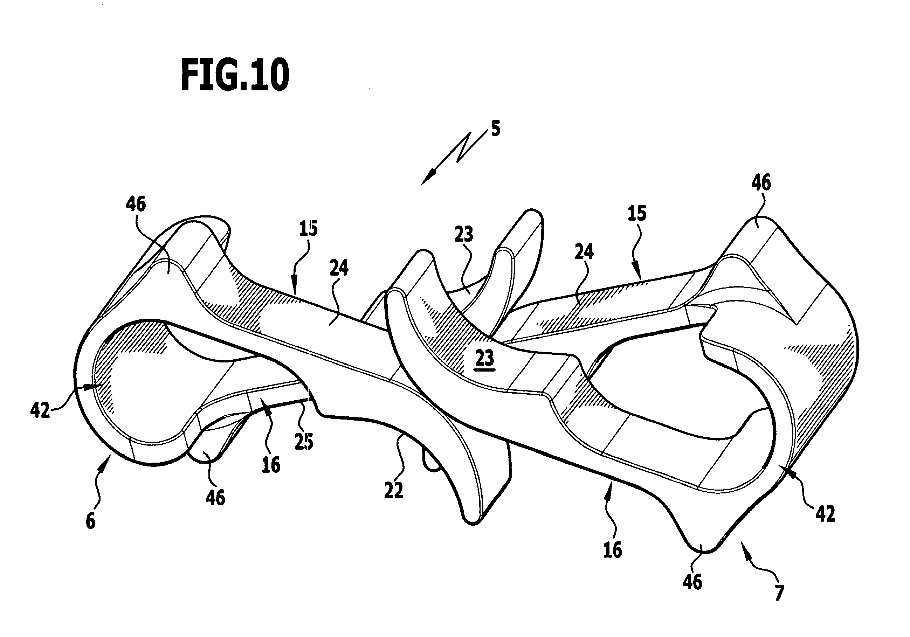

Die Implantatkomponenten des Implantates der Figuren 10 bis 11 sind ähnlich aufgebaut wie bei dem Ausführungsbeispiel der Figuren 7 bis 9, jedoch weisen diese Implantatkomponenten nur zwei einteilige Stützarme 15, 16 auf, die seitlich so versetzt an dem Brückenabschnitt 42 angeordnet sind, dass sie nebeneinander liegen, das heißt in diesem Falle ist kein in Längsrichtung geschlitzter Stützarm vorgesehen, wie bei dem Ausführungsbeispiel der Figuren 7 bis 9. Bei diesem Ausführungsbeispiel bilden außerdem die etwas verdickten Enden der Stützarme selbst die Stützflächen 22, 23 aus, im Bereich der freien Enden sind diese Stützarme 15, 16 dabei stärker abgebogen als dies bei dem Ausführungsbeispiel der Figuren 7 bis 9 der Fall ist, so dass beim Zusammenschieben auf jeden Fall ein Aufgleiten erleichtert wird.

Die Außenflächen der Stützarme 15, 16 sind im mittleren Bereich ebenfalls eben ausgebildet, es schließt sich aber an diesen ebenen Bereich jeweils eine Erhebung 46 an, die dazu führt, dass das abgebogene Ende der Stützarme beim Erreichen dieser Erhebung 46 sehr viel stärker abgebogen wird als längs der schräg verlaufenden, ebenen Außenflächen der Stützarme 15, 16. Dies begrenzt einmal das Zusammenschieben, zum anderen wird bei weiterem Zusammenschieben eine besonders starke Veränderung des Abstandes der Stützflächen erreicht, das heißt der Operateur spürt beim Zusammenschieben einen erhöhten Widerstand und kann daher beurteilen, wie weit die Implantatkomponenten zusammengeschoben sind.

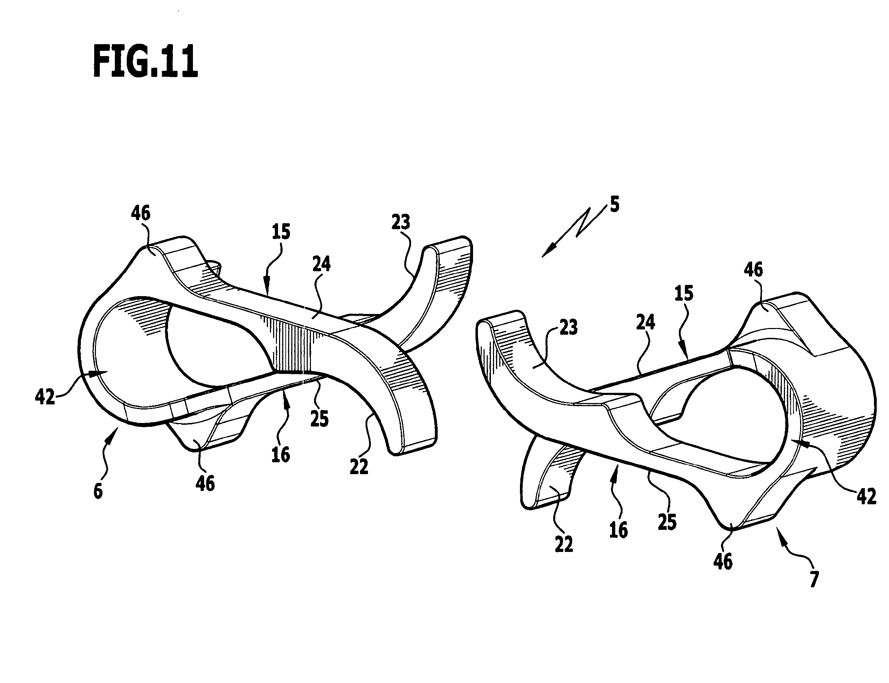

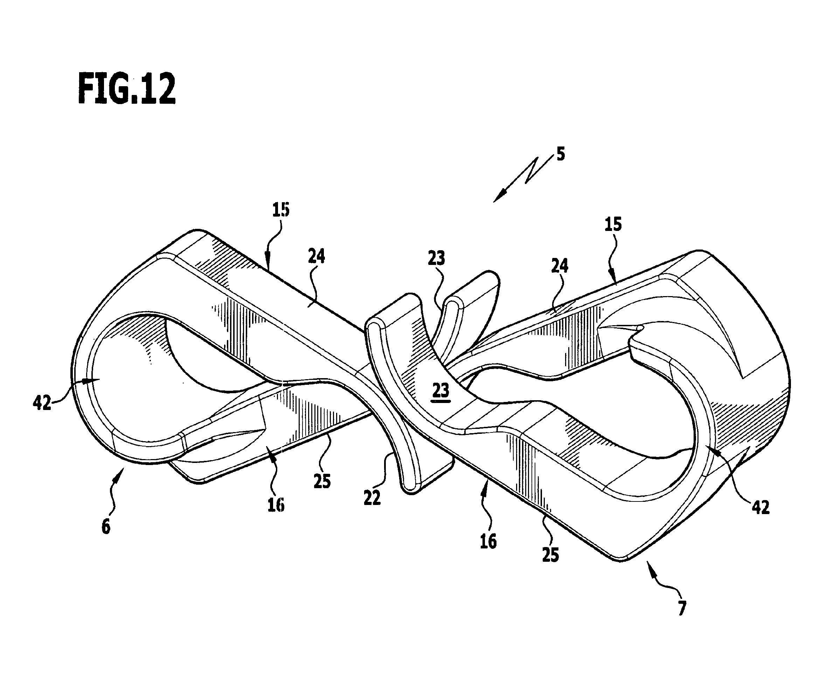

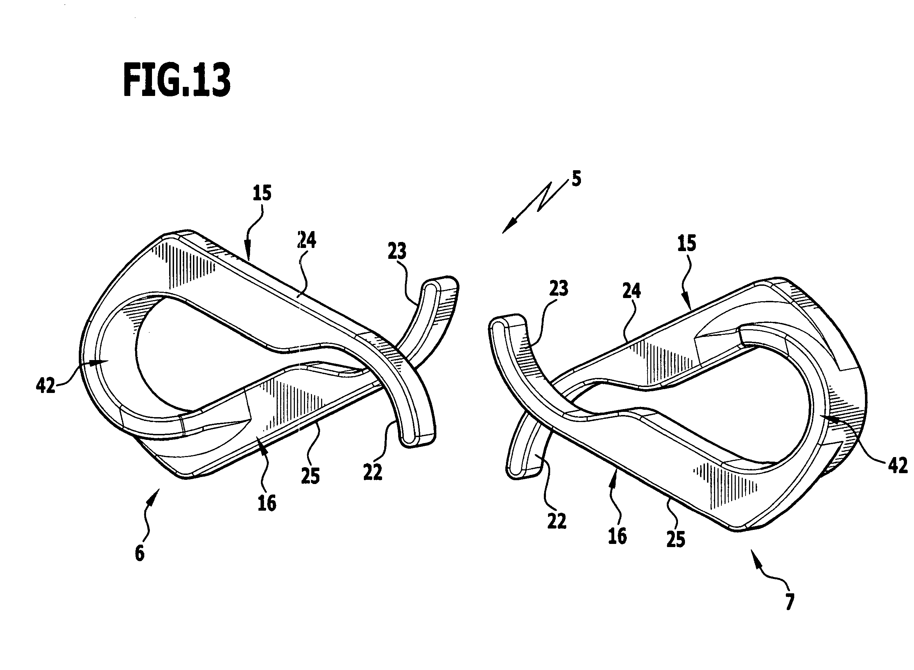

Bei dem Ausführungsbeispiel der Figuren 12 und 13 sind die Implantatkomponenten denen des Ausführungsbeispiels der Figuren 10 und 11 sehr ähnlich ausgebildet, die Stützarme sind jedoch verdickt, wobei die Stützflächen 22, 23 unmittelbar in diese verdickten Schlitzarmen eingeformt sind. Außerdem fehlen bei diesem Ausführungsbeispiel die Erhebungen 46 auf den Außenflächen.

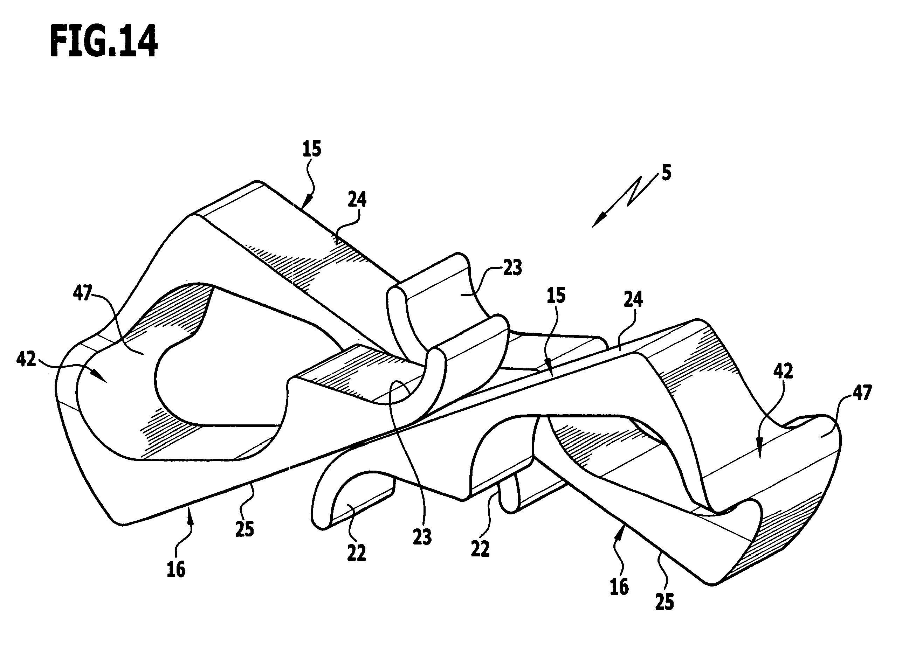

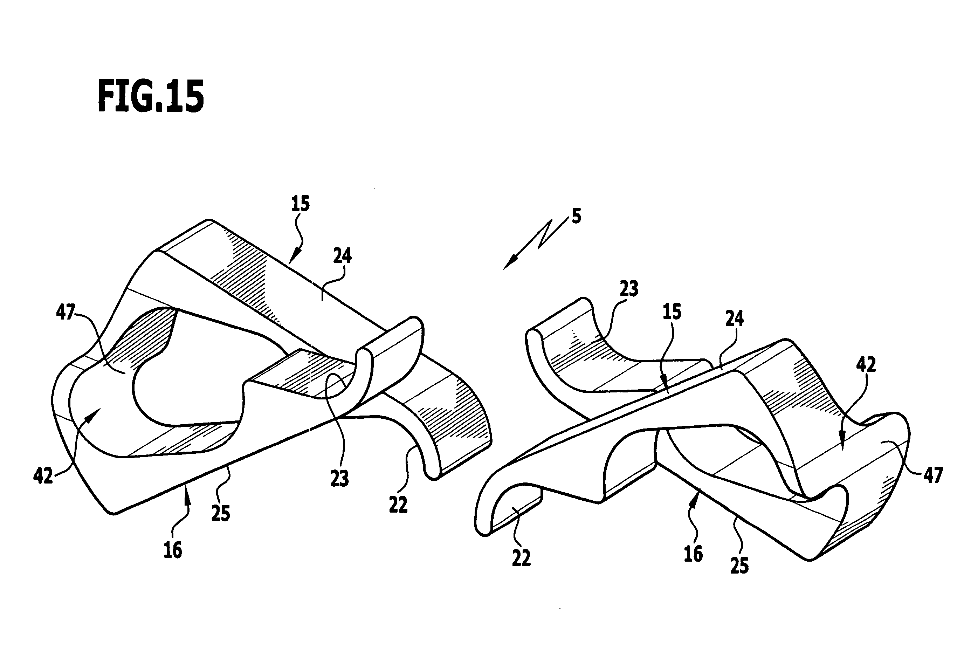

Bei dem Ausführungsbeispiel der Figuren 14 und 15 ist eine ähnliche Ausgestaltung gewählt, wie bei dem Ausführungsbeispiel der Figuren 10 und 11. Der bandförmige Brückenabschnitt 42 ist bei diesem Ausführungsbeispiel ähnlich schmal ausgebildet, wie die sich anschließenden Stützarme, das heißt es ergibt sich nur ein sehr schmaler Verbindungsbereich 47, über den die nebeneinander liegenden Stützarme miteinander verbunden sind. Auch bei dieser Ausführungsform fehlen die Erhebungen 46.

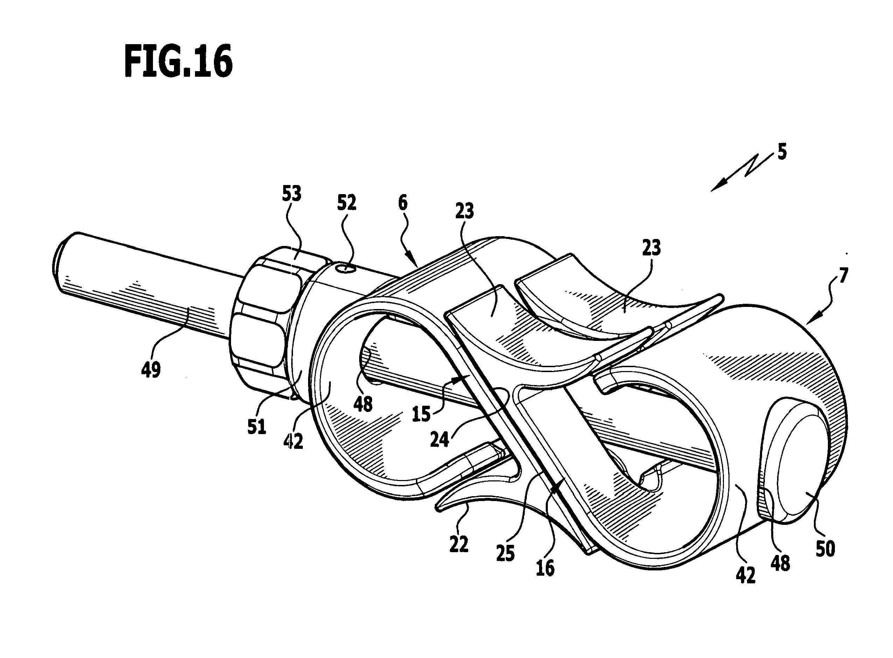

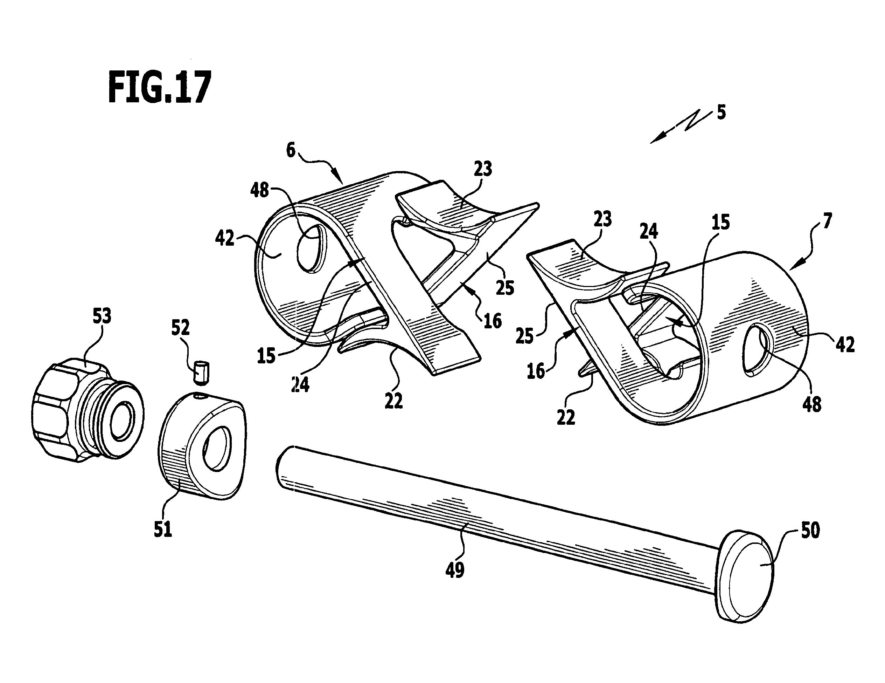

Bei dem Ausführungsbeispiel der Figuren 16 und 17 sind beide Implantatkomponenten ähnlich ausgebildet wie bei dem Ausführungsbeispiel der Figuren 10 und 11, dabei sind die Stützarme ebenso wie der bandförmige Brückenabschnitt 42 als dünne Stege ausgebildet, die an ihrem Ende nach außen abstehende Vorsprünge 20, 21 tragen mit entsprechenden Stützflächen 22 beziehungsweise 23. In sofern ist eine ähnliche Ausgestaltung gegeben wie bei dem Ausführungsbeispiel der Figuren 1 bis 6, jedoch weist jede Implantatkomponente nur zwei nebeneinander liegende Stützarme auf.

In beiden Implantatkomponenten ist im stegförmigen Brückenabschnitt 42 jeweils eine Durchbrechung 48 angeordnet, und durch diese Durchbrechungen 48 ragt ein einziger Zuganker 49 hindurch, der zwischen den beiden Stützarmen 15 und 16 der Implantatkomponenten hindurchtritt und somit beide Implantatkomponenten mittig durchsetzt. Der Zuganker 49 stützt sich mit einem verdickten Kopf 50 an einer Implantatkomponente ab, auf das gegenüberliegende Ende ist eine Haltemuffe 51 aufgeschoben, die mittels einer in eine Gewindebohrung der Haltemuffe 51 eingeschraubten Schraube 52 längs des Zugankers 49 festgelegt werden kann. Weiterhin ist auf den Zuganker 49 ein Griffteil 53 aufgeschoben, mit welchem die Haltemuffe 51 gegen den Kopf 50 geschoben werden kann, solange die Schraube 52 noch nicht festgeschraubt ist. Dadurch können die beiden Implantatkomponenten gegeneinander geschoben werden, so dass die Stützflächen 22 und 23 in der beschriebenen Weise durch Aufgleiten der Stützarme 15, 16 aufeinander nach oben und unten ausgefahren werden.

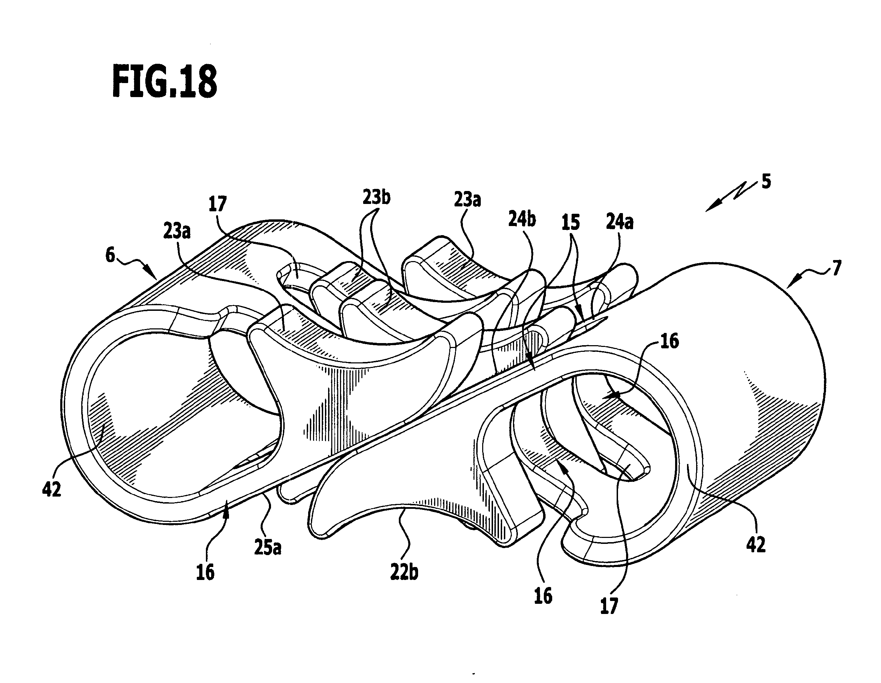

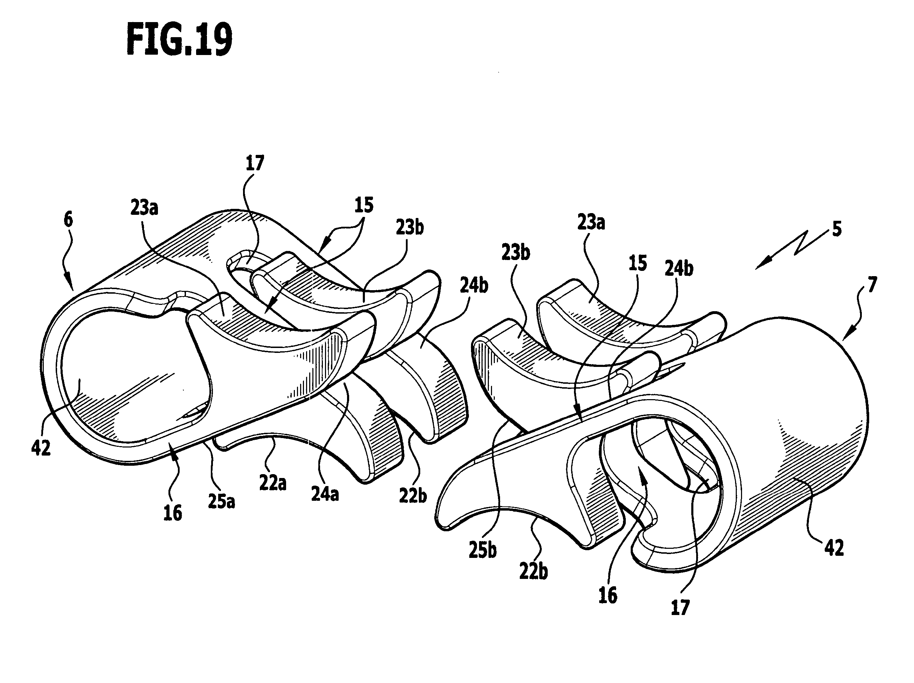

Bei dem Ausführungsbeispiel der Figuren 18 und 19 ist eine ähnliche Ausgestaltung gewählt wie bei dem Ausführungsbeispiel der Figuren 7 bis 9, jedoch sind jeweils beide Stützarme 15, 16 einer Implantatkomponente durch einen Längsschlitz 17 in nebeneinander liegende Stützarmabschnitte 18, 19 unterteilt, das heißt damit sind auch jeweils alle Stützflächen doppelt ausgebildet, die einzelnen Stützarmabschnitte und Stützflächen der beiden Implantatkomponenten greifen dabei kammartig ineinander.

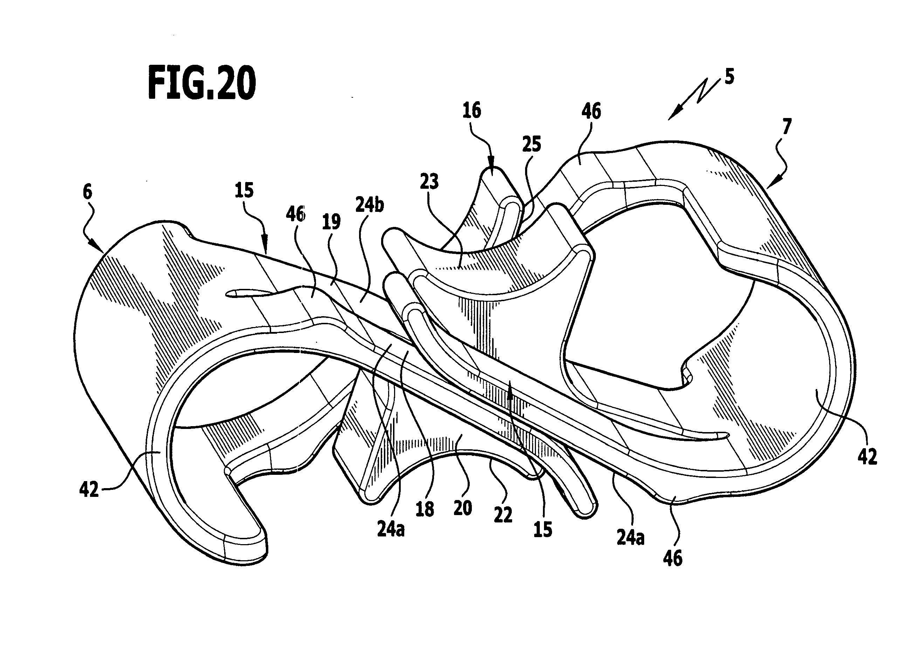

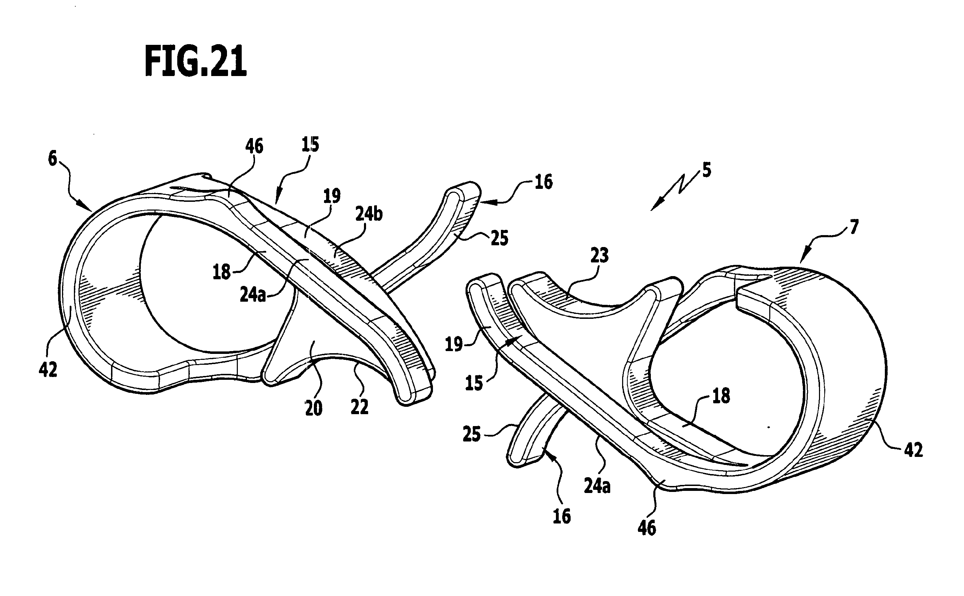

Bei dem Ausführungsbeispiel der Figuren 20 und 21 weist jede Implantatkomponente einen in Längsrichtung geschlitzten Stützarm 15 und einen nicht geteilten Stützarm 16 auf, dabei liegen die beiden Stützarmabschnitte 18, 19 des geschlitzten Stützarmes 15 unmittelbar nebeneinander. Der innen liegende Stützarmabschnitt 19 des geteilten Stützarmes 15 trägt in ähnlicher Weise, wie dies beim Ausführungsbeispiel der Figuren 1 bis 9 beschrieben ist, einen Vorsprung 20 mit einer Stützfläche 22, dies ist aber der einzige Vorsprung mit Stützfläche dieser Art an der Implantatkomponente, der Stützarmabschnitt 18 und der zweite Stützarm 16 sind nicht mit einem solchen Vorsprung und einer solchen Stützfläche versehen. Der Stützarmabschnitt 18 und der Stützarm 16 sind über die gesamte Länge als dünner Steg ausgebildet, dessen Dicke etwa der Dicke des stegförmigen Brückenabschnittes 42 entspricht. Der Stützarmabschnitt 19 mit dem Vorsprung 20 und der Stützfläche 22 befindet sich in der Mitte zwischen dem Stützarmabschnitt 18 und dem Stützarm 16, dabei sind die beiden außen liegenden Stützarme 16 und Stützarmabschnitt 18 gleich ausgebildet mit einer im Wesentlichen ebenen Außenfläche 24a beziehungsweise 25, die in ähnlicher Weise wie beim Ausführungsbeispiel der Figuren 10 und 11 in eine Erhebung 46 übergehen. Beim Zusammenschieben der beiden Implantatkomponenten werden in gleicher Weise wie dies bei den anderen Ausführungsbeispielen erörtert worden ist, die Stützarme an ihren Außenflächen aneinander aufgleiten und dadurch stärker überkreuzt werden. Dies führt einerseits in gleicher Weise zur Entfernung der beiden Stützflächen 22, 23 voneinander, andererseits werden dadurch aber auch die relativ flexiblen Stützarmabschnitte 18 und Stützarm 16 nach außen abgebogen, und zwar wegen ihrer größeren Flexibilität auch relativ stark. Die freien Enden des Stützarmes 16 und des Stützarmabschnittes 18 werden dabei zu beiden Seiten der jeweiligen Stützfläche 22 beziehungsweise 23 aufgebogen und bilden somit zu beiden Seiten der Stützflächen vorstehende Anlageelemente aus, die ähnlich wie die Anlageelemente 37 und 38 bei den vorstehend beschriebenen Ausführungsbeispielen seitlich an den Dornfortsätzen zur Anlage kommen und dadurch das Implantat gegen seitliche Verschiebung sichern. Bei diesem Ausführungsbeispiel sind also durch unterschiedliche Ausgestaltung der Stützarme sowohl die Stützarme mit abstandsveränderlichen Stützflächen als auch die Anlageelemente zur seitlichen Anlage an den Dornfortsätzen in einem einzigen Bauteil zusammengefasst, nämlich der jeweiligen Implantatkomponente 6, 7.

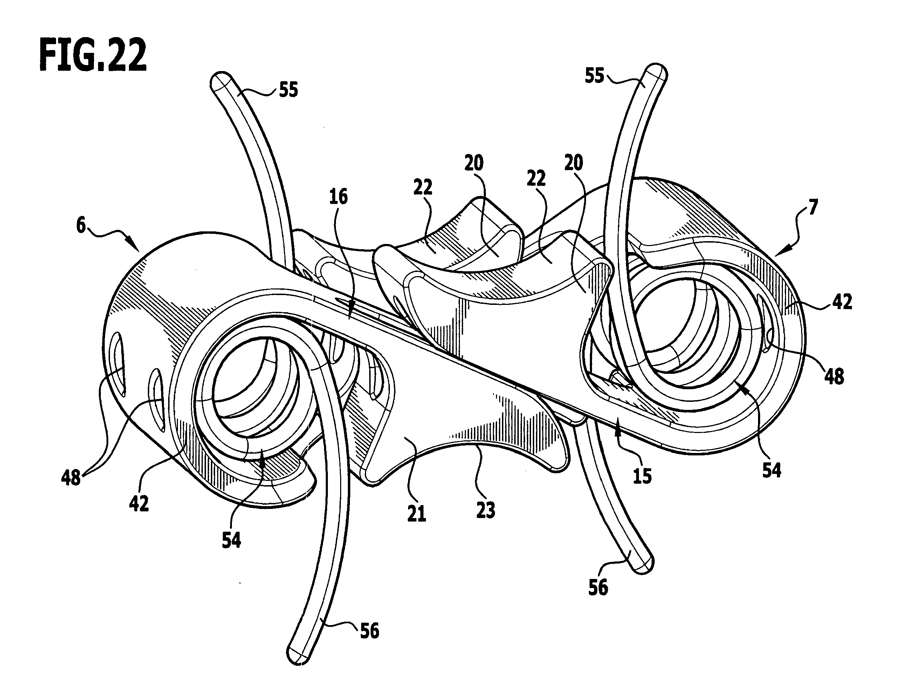

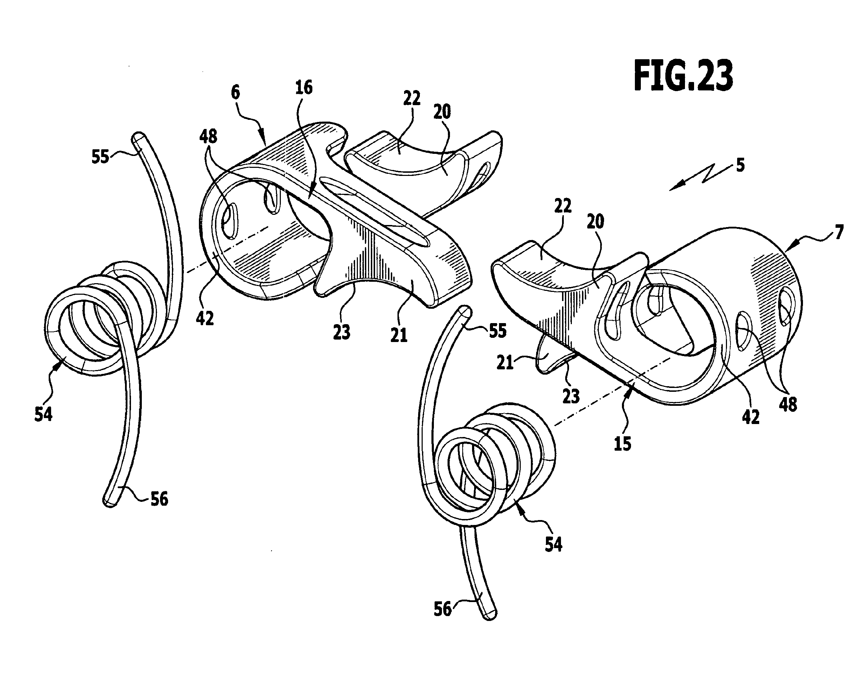

Das in den Figuren 22 und 23 dargestellte Ausführungsbeispiel ist ähnlich aufgebaut wie das Ausführungsbeispiel der Figuren 16 und 17. Beide Implantatkomponenten tragen ungeschlitzte Stützarme 15, 16 mit jeweils einem Vorsprung 20, 21 und einer Stützfläche 22, 23. Um die Implantatkomponenten 6, 7 gegeneinander zu verschieben, sind in beiden Implantatkomponenten sowohl in dem Brückenabschnitt 42 als auch in den Vorsprüngen 20, 21 der Stützarme 15, 16 Durchbrechungen vorgesehen, durch die zwei nebeneinander liegende Zuganker hindurchgeführt werden können, die in der Zeichnung aus Gründen der Übersichtlichkeit nicht gesondert dargestellt sind.

Außerdem ist in den Innenraum jeder Implantatkomponente 6, 7, also in den einerseits vom Brückenabschnitt 42 und andererseits von den Stützarmen 15, 16 bis zum Überkreuzungspunkt eingeschlossenen Raum, eine Schraubenfeder 54 eingesetzt, deren Längsachse quer zu den Stützarmen verläuft, so dass sich die Schraubenfeder mit ihrem Umfang an die Innenseite des gebogenen Brückenabschnittes 42 anlegt. Die freien Enden 55, 56 der Schraubenfeder 54 ragen neben den Stützarmen 15, 16 nach außen und stehen etwa quer zur Längsrichtung der Stützarme nach außen ab, und zwar zu beiden Seiten der Stützflächen 22, 23. Sie bilden auf diese Weise Anlageelemente zur Festlegung des Implantates an den Seitenflächen der Dornfortsätze. Beim Implantieren des Implantates können die freien Enden aufgrund der Federwirkung der Schraubenfeder 54 so abgebogen sein, dass sie etwa in Längsrichtung der Stützarme verlaufen und nicht nach außen abstehen, so dass die Bauhöhe gering bleibt und die freien Enden das Einführen nicht behindern. Sobald das Implantat zwischen die Dornfortsätze eingesetzt ist, können die freien Enden freigegeben werden und federn dann in die in Figur 22 gezeigte Position, bei der sie zu beiden Seiten der Stützfläche Anlageelemente für den Dornfortsatz ausbilden.

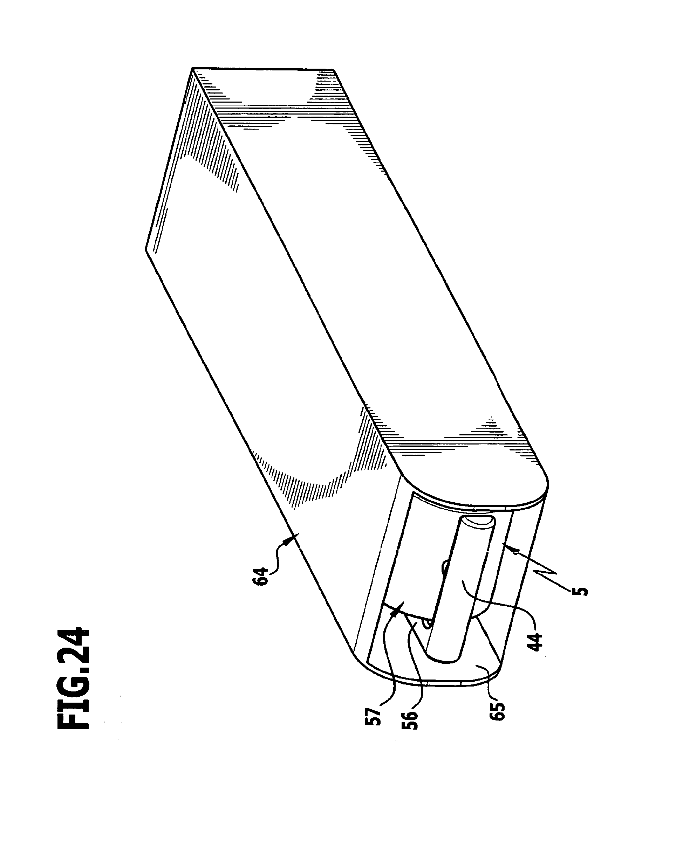

Bei dem Ausführungsbeispiel der Figuren 24 bis 28 sind die beiden Implantatkomponenten ähnlich ausgebildet wie im Ausführungsbeispiel der Figuren 22 und 23, es fehlen lediglich die Durchbrechungen im Brückenabschnitt und in dem Vorsprüngen zur Durchführung von Zugankern.

Jede Implantatkomponente 6, 7 wird umgeben von einer Klammer 57, 58, diese sind gleich aufgebaut, nachstehend wird daher nur eine der beiden Klammern 57 näher beschrieben. Diese Klammer umfasst einen stegförmigen, gebogenen Mittelabschnitt 59, der sich außenseitig flächig an den gebogenen Brückenabschnitt 42 der Implantatkomponente 6, 7 anlegt und der sich über einen Umfangsbereich erstreckt, der größer ist als 180°, so dass dieser Mittelabschnitt 59 auf der Implantatkomponente dadurch gehalten ist, dass er den Brückenabschnitt 42 über einen Winkelbereich von mehr als 180° umgibt. Der Mittelabschnitt 59 kann elastisch aufbiegbar sein, so dass die Klammer 57 auf dem Brückenabschnitt 42 unter Aufweitung des Mittelabschnittes 59 aufgeschoben werden kann und in der aufgeschobenen Position durch eine Verengung des Mittelabschnittes 59 in dieser Position festgelegt wird.

An seinen Enden trägt der Mittelabschnitt 59 an beiden Seiten einen im wesentlichen streifenförmigen, nach außen gebogenen Steg 60, 61, diese Stege sind seitlich neben den beiden Stützarmen 15, 16 der Implantatkomponenten 6, 7 angeordnet, so dass die Stützflächen 22, 23 der Implantatkomponenten 6,7 zwischen diesen Stegen 60, 61 liegen. Die nach außen gebogenen Stege 60, 61 der auf die Implantatkomponenten 6, 7 aufgesetzten Klammern 57, 58 liegen mit ihren Innenseiten an Aufgleitflächen 62, 63 an, die sich an der Außenseite der Brückenabschnitte 42 der beiden Implantatkomponenten 6, 7 befinden, und zwar unmittelbar neben dem Austritt des jeweiligen Stützarmes 15, 16 aus diesem Brückenabschnitt 42. Beim Zusammenschieben der beiden Implantatkomponente 6, 7 werden dadurch die beiden Stege 60, 61, die sich im Gegensatz zu den Stützarmen 15, 16 nicht überkreuzen, nach außen verschwenkt und bilden zu beiden Seiten der Stützflächen 22, 23 seitliche Anlageelemente aus, die sich an der Seitenfläche des Dornfortsatzes anlegen.

Bei dieser Ausführungsform werden also die Anlageelemente als separate Teile ausgeführt, die an den auf die Implantatkomponenten 6, 7 aufgeschobenen Klammern 57, 58 angeordnet sind und die durch Aufgleiten an der Außenseite der gegenüberliegenden Implantatkomponente in ihre Anlageposition ausgeschwenkt werden.

Auf diese Weise kann ein Implantat wahlweise entweder ohne diese Klammern 57, 58 oder mit diesen Klammern 57, 58 eingesetzt werden, der Operateur kann kurzfristig entscheiden, ob die Klammern 57, 58 aufgesetzt werden und dadurch Anlageelemente geschaffen werden, oder ob dies nicht notwendig ist.

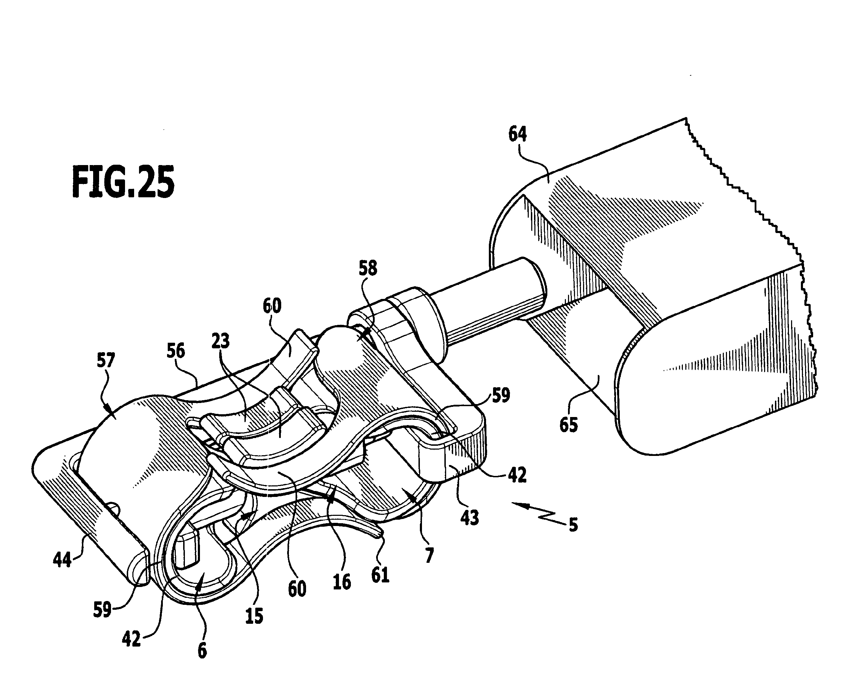

Um die Implantatkomponenten zusammen zu schieben, können an der Außenseite der Klammern 57, 58 Endstücke 43, 44 anliegen, von denen eines mit einem Zuganker 56 einstückig verbunden ist, während das andere auf diesem Zuganker längsverschieblich ist (Figur 25). In sofern ergibt sich eine Konstruktion, die ähnlich ist wie die Konstruktion beim Ausführungsbeispiel der Figuren 7 bis 9, mit dem Unterschied, dass ein Zuganker nur einseitig am Implantat angeordnet ist.

Bei dem Ausführungsbeispiel der Figuren 24 bis 28 ist außerdem ein längliches, quaderförmiges Gehäuse 64 dargestellt mit einer Öffnung 65 an einer Stirnseite, in die das Implantat der Figuren 25 bis 28 eingeschoben ist. Auf diese Weise wird das Implantat in dem Gehäuse 64 aufbewahrt und kann in dieser Form auch implantiert werden. Das Gehäuse schützt dabei sowohl das Implantat als auch das umgebende Gewebe und kann nach dem Einsetzen des Implantates von diesem abgezogen werden, dabei tritt das Implantat in der aus Figur 25 ersichtlichen Weise aus dem Innenraum des Gehäuses 64 heraus und kann dann in der vorstehend beschriebenen Weise durch Annäherung der Endstücke 43, 44 in die endgültige Position gebracht werden, in der der Abstand der Stützflächen 22 und 23 eingestellt wird und in der gleichzeitig die Stege 60, 61 nach außen abgebogen werden.

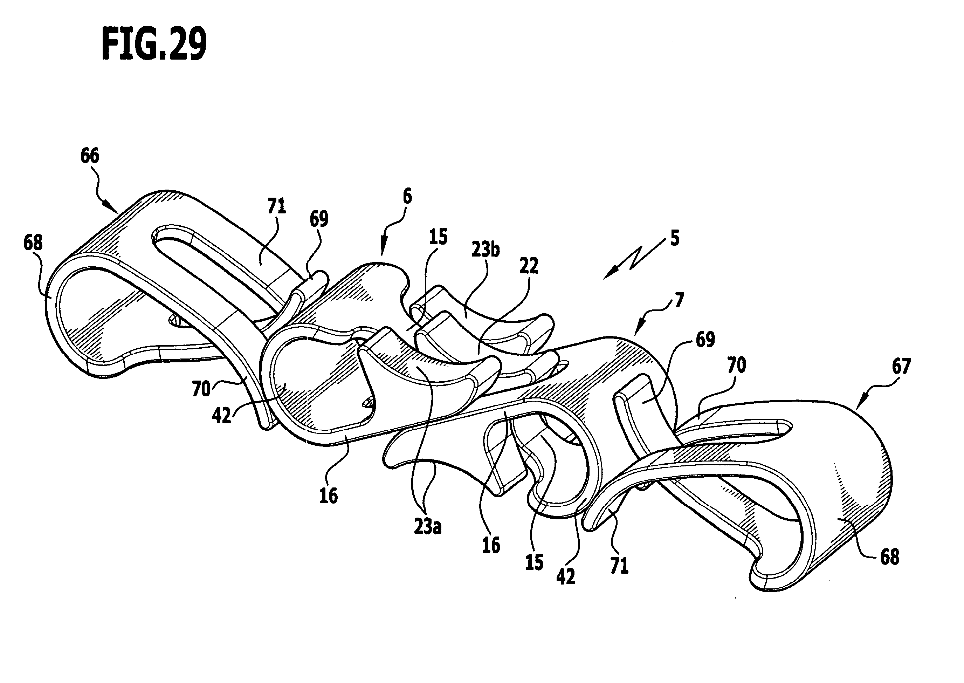

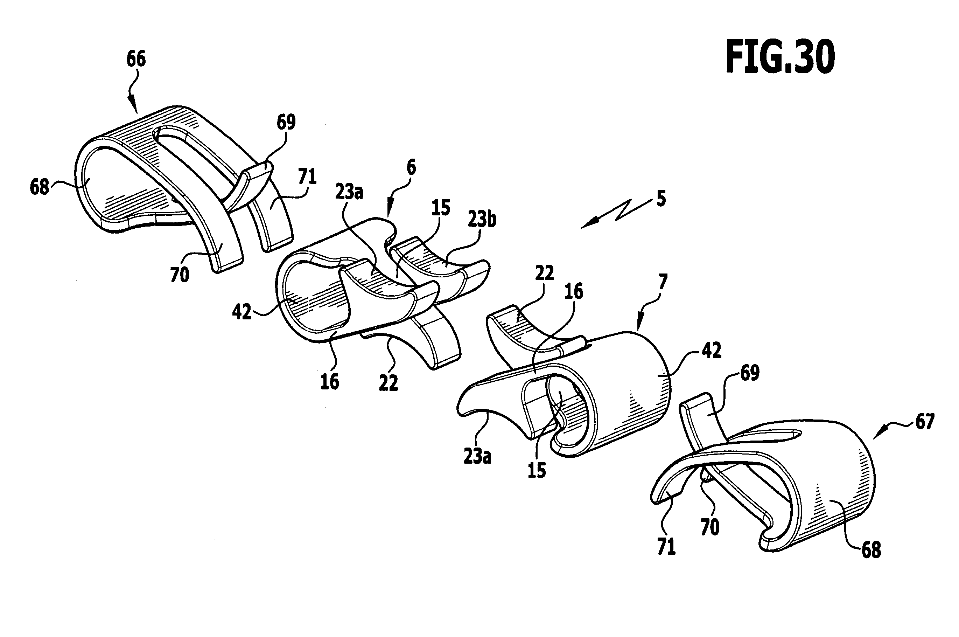

Bei dem Ausführungsbeispiel der Figuren 29 und 30 sind die Implantatkomponenten 6, 7 aufgebaut wie im Ausführungsbeispiel der Figuren 7 bis 9. Zusätzlich zu den Implantatkomponenten finden ähnlich aufgebaute Schubklammern 66, 67 Anwendung, die gleich aufgebaut sind und von denen daher nur die Schubklammer 66 näher erläutert wird.

Diese Schubklammer 66 ist bandförmig aufgebaut mit einem gebogenen, bandförmigen Brückenabschnitt 68, der an einem Ende einen mittigen, im wesentlichen geradlinigen Steg 69 und am anderen Ende zwei parallel und im Abstand zueinander verlaufende Stege 70, 71 trägt, wobei der mittige Steg 69 in den Zwischenraum 72 zwischen den Stegen 70, 71 eintaucht, so dass sich die Stege 79 einerseits und die Stege 70 und 71 andererseits überkreuzen. Alle Stege 69, 70, 71 sind an ihren freien Enden zur Außenseite hin abgebogen und liegen mit ihren jeweiligen Außenflächen an der Außenseite des Brückenabschnittes 42 je einer Implantatkomponente 6, 7 an. Es erfolgt somit eine Hintereinanderanordnung der beiden Implantatkomponenten 6, 7 und auf gegenüberliegenden Seiten der Schubklammern 66, 67.

Durch aus der Zeichnung nicht ersichtliche Mittel können die Schubklammern 66, 67 gegeneinander geschoben werden, dabei schieben sie einmal die Implantatkomponenten 6, 7 gegeneinander, so dass dadurch in der vorstehend beschriebenen Weise die Stützflächen 22 und 23 voneinander entfernt werden, zum anderen werden aber auch die an den Schubklammern 66, 67, angeordneten, relativ flexiblen Stege 69, 70,71 stärker überkreuzt und so verbogen, dass sie nach dem Zusammenschieben zu beiden Seiten der Stützflächen 22, 23 nach oben und unten von dem Implantat abstehen und Anlageelemente ausbilden, die sich seitlich an die Dornfortsätze anlegen können.

Auch bei diesem Implantat ist es daher möglich, das Implantat entweder ohne die Schubklammern 66, 67 oder zusätzlich mit den Schubklammern 66, 67 zu implantieren, bei Verwendung der Schubklammern 66, 67 bilden diese einmal die Elemente aus, mit denen die Implantatkomponenten 6, 7 gegeneinander geschoben werden, zum anderen formen sich dabei aber auch zu beiden Seiten der Stützflächen die seitlichen Anlageelemente. Schubklammern 66, 67 dieser Art können auch bei anderen Ausgestaltungen der Implantatkomponenten 6, 7 Verwendung finden.

Die vorstehend beschriebenen Ausführungsformen zeichnen sich dadurch aus, dass die Stützarme einer Implantatkomponente sich überkreuzen und durch Zusammenschieben von zwei Implantatkomponenten die Überkreuzung noch verstärkt wird, die Stützarme also an ihren freien Enden aufgespreizt werden. Dem entsprechend sind die Stützflächen jeweils an den Innenseiten der Stützarme angeordnet und gegenüber den Stützarmen so geneigt, dass die Stützflächen an der Oberseite und an der Unterseite des Implantates im Wesentlichen parallel zueinander und parallel zur Verschiebeebene der Implantatkörper angeordnet sind.

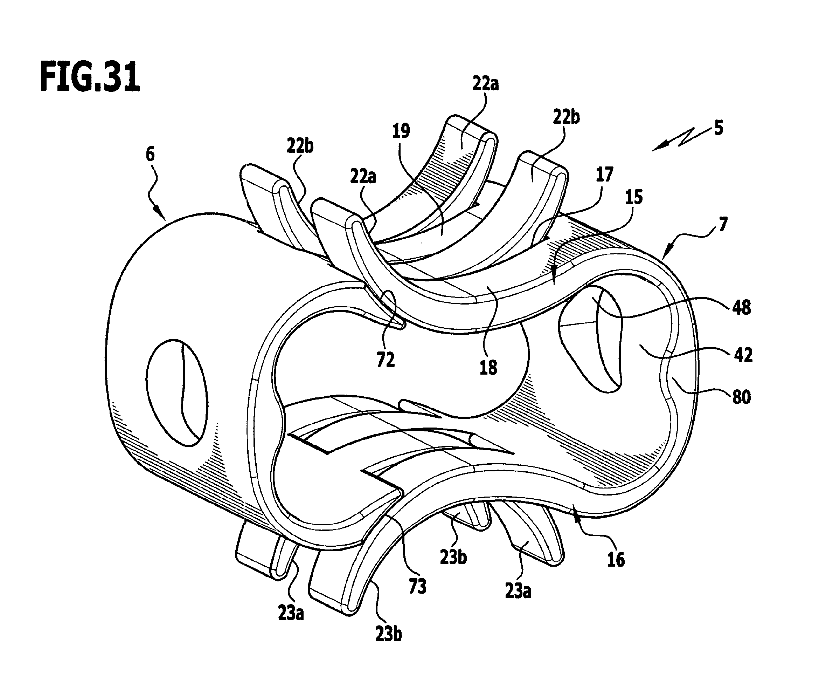

Bei den nachstehen an Hand der Figuren 31 bis 44 beschriebenen Ausführungsformen ist eine andere Konstruktion der Implantatkörper 6, 7 gewählt, die aber trotzdem so ähnlich aufgebaut sind, dass einander entsprechende Teile dieselben Bezugszeichen tragen. Im Gegensatz zu den vorstehend beschriebenen Ausführungsbeispielen überkreuzen sich bei diesen Ausführungsformen die Stützarme nicht, sondern sie verlaufen im Wesentlichen parallel zueinander und parallel zu der Verschiebeebene der Implantatkomponenten, das heißt die Implantatkomponenten 6, 7 haben im Wesentlichen die Form eines U, wobei die Stützarme etwa parallel verlaufende Schenkel ausbilden und an einem Ende über einen Brückenabschnitt 42 verbunden sind.

Während die Stützarme bei den Ausführungsbeispielen der Figuren 1 bis 30 mit ihren Außenflächen an den Außenflächen der Stützarme des jeweils anderen Implantakörpers anliegen und dadurch beim Zusammenschieben noch stärker überkreuzt werden, liegen die Stützarme bei den Ausführungsbeispielen der Figuren 31 bis 44 mit ihren Innenseiten an Aufgleitflächen 72, 73 der jeweils anderen Implantatkomponente an, diese Aufgleitflächen 72, 73 sind jeweils im Übergangsbereich zwischen dem Brückenabschnitt 42 und den Stützarmen 15, 16 einer Implantatkomponente angeordnet, und zwar neben den Stützarmen der die Aufgleitfläche tragenden Implantatkomponente.

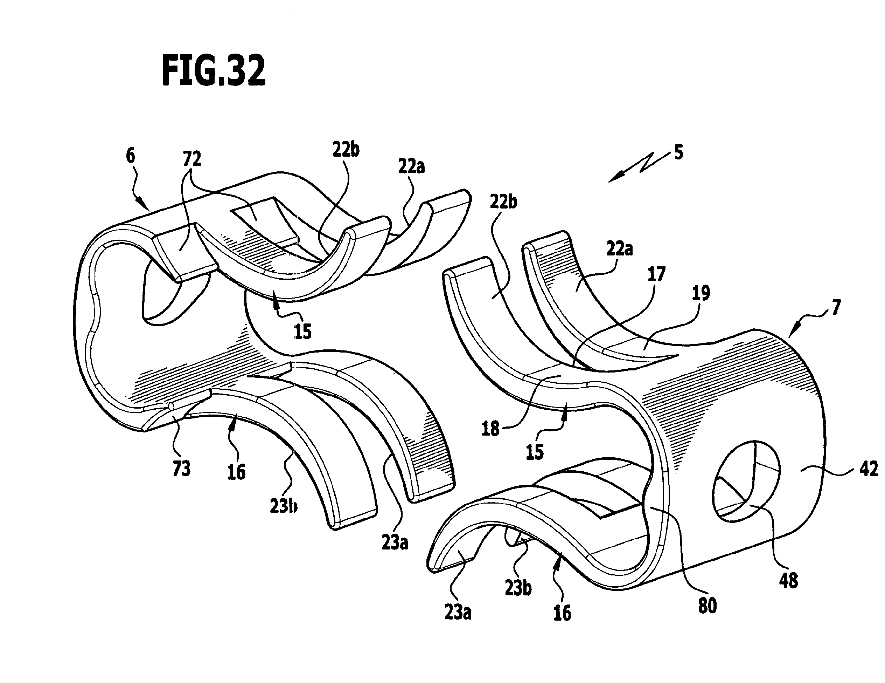

Beim Ausführungsbeispiel der Figuren 31 und 32 sind die Stützarme 15, 16 jeweils durch einen Längsschlitz 17 in Stützarmabschnitte 18, 19 unterteilt, so dass insgesamt auf der Oberseite und auf der Unterseite des Implantates jeweils vier kammartig ineinander greifende Stützarmabschnitt 18. 19 angeordnet sind, die sich mit ihren Innenflächen jeweils an einer Aufgleitfläche 72, 73 der jeweils anderen Implantatkomponente abstützen und die dadurch beim Zusammenschieben der Implantatkomponenten 6, 7 nach außen abgebogen werden, das heißt die Stützarme 15, 16 jeder Implantatkomponente 6, 7 werden beim Zusammenschieben der Implantatkomponenten in ihrer gesamten Länge nach außen geschwenkt oder aufgespreizt, so dass der Abstand ihrer Außenflächen vergrößert wird.

Die Stützarme sind dabei in allen Fällen konkav gebogen, so dass sich an den Außenflächen der Stützarme durch diese konkav gebogene Stützflächen 22, 23 ergeben, deren Abstand durch das Zusammenschieben der Implantatkomponenten 6, 7 vergrößert werden kann.

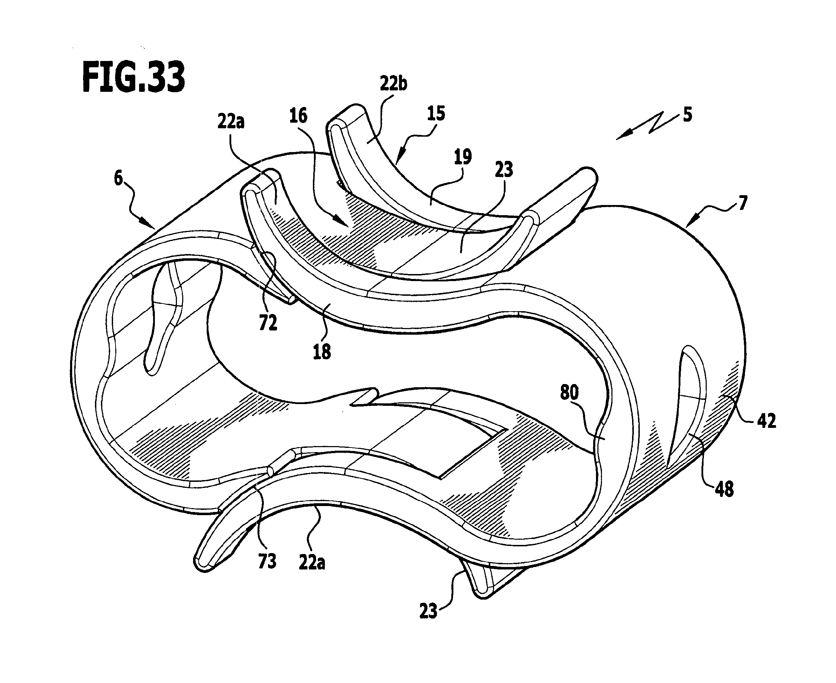

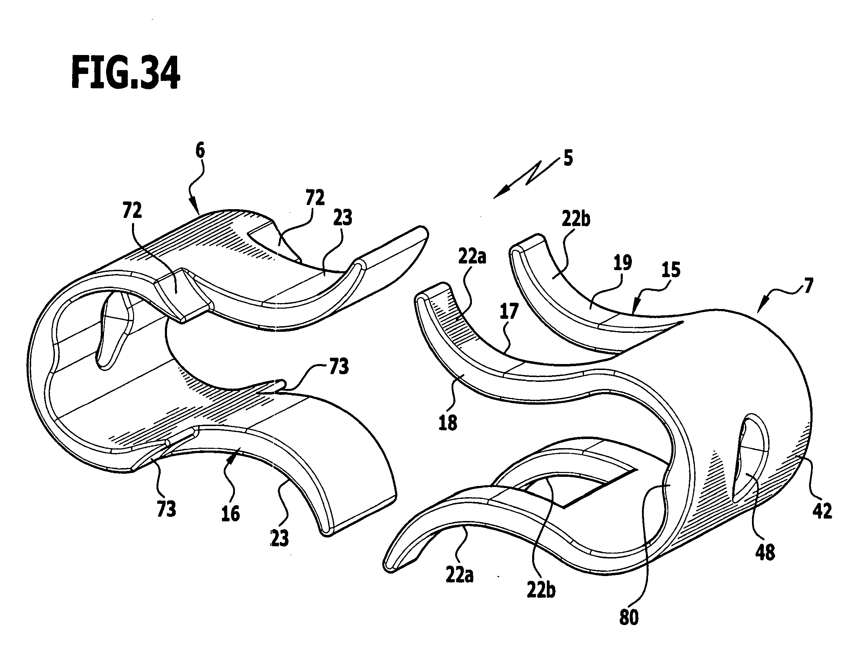

Während bei dem Ausführungsbeispiel der Figuren 31 und 32 die Stützarme auf der Oberseite und der Unterseite beider Implantatkomponenten paarweise ausgebildet sind, so dass die Implantatkomponenten 6, 7 zueinander gleich aufgebaut sind, werden bei dem Implantat der Figuren 33 und 34 zwei unterschiedlich aufgebaute Implantatkomponente 6, 7 eingesetzt, nämlich eine erste Implantatkomponente 6, die an der Oberseite und an der Unterseite jeweils nur einen mittigen Stützarm 15 trägt, sowie eine zweite Implantatkomponente 7, die an der Oberseite und an der Unterseite zwei parallel zueinander verlaufende, einen Abstand zwischen sich ausbildende und den Stützarm 15 der anderen Implantatkomponente 5 zwischen sich aufnehmende Stützarmabschnitte 18, 19 trägt.

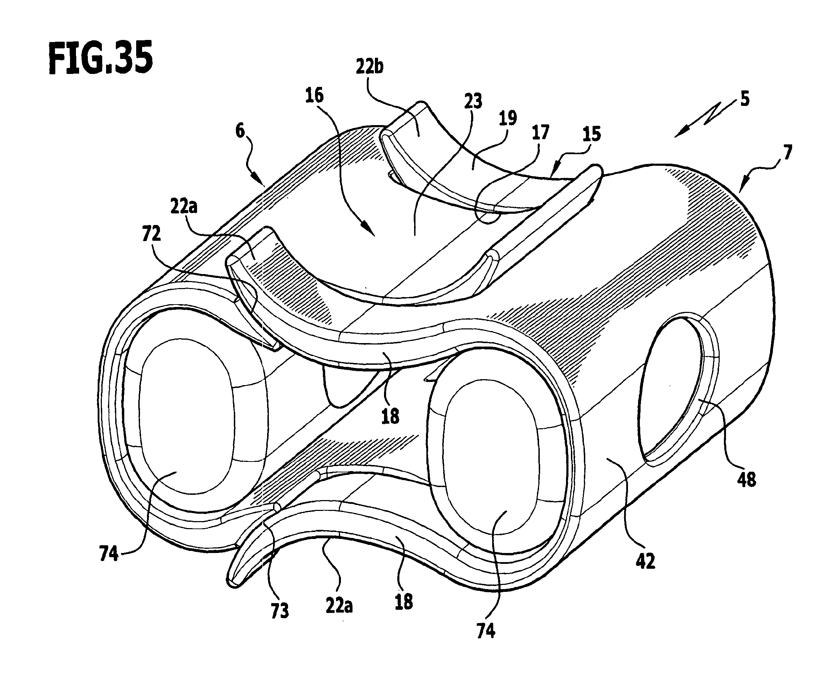

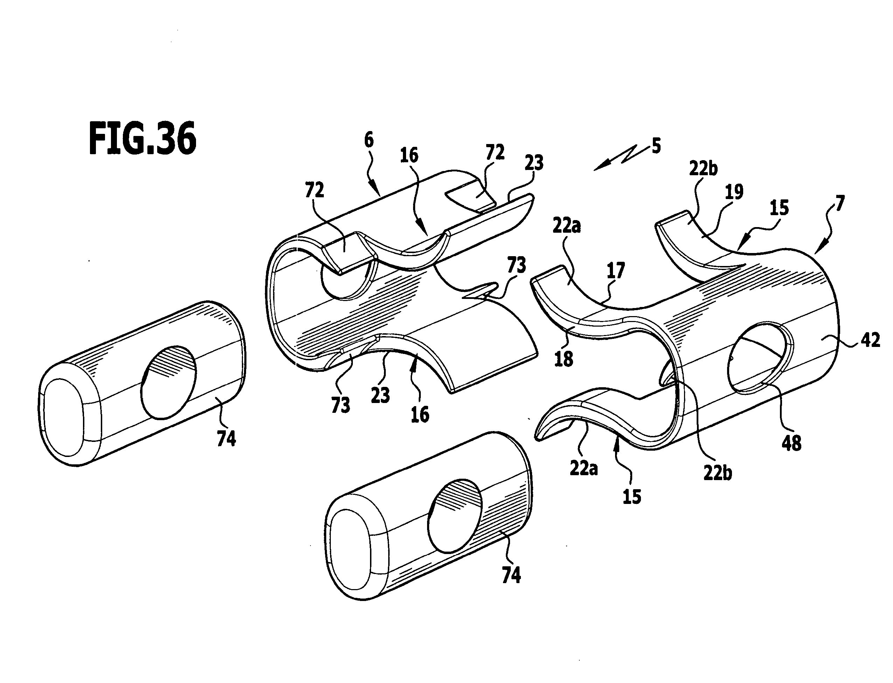

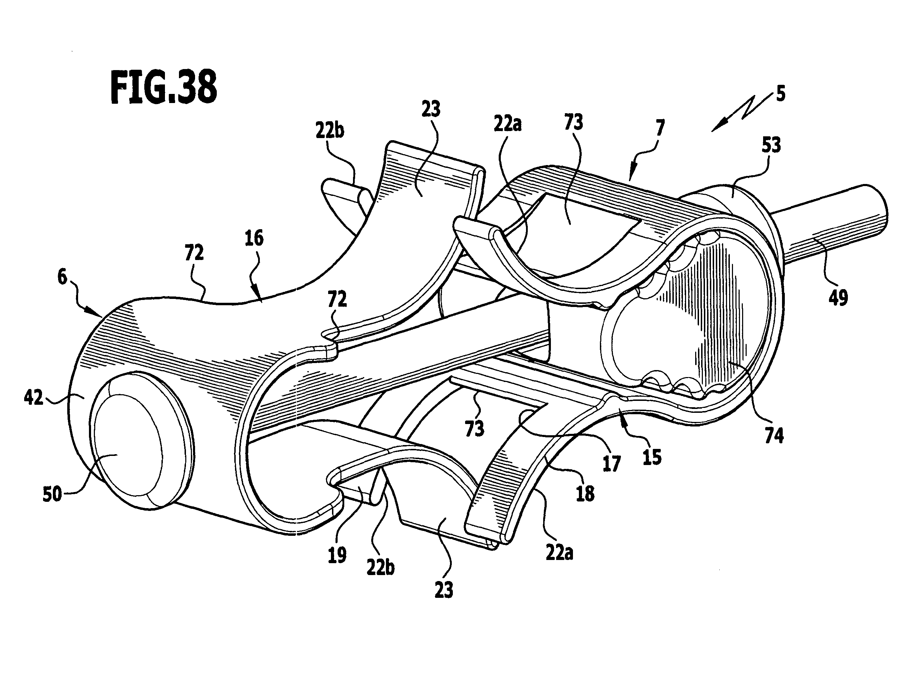

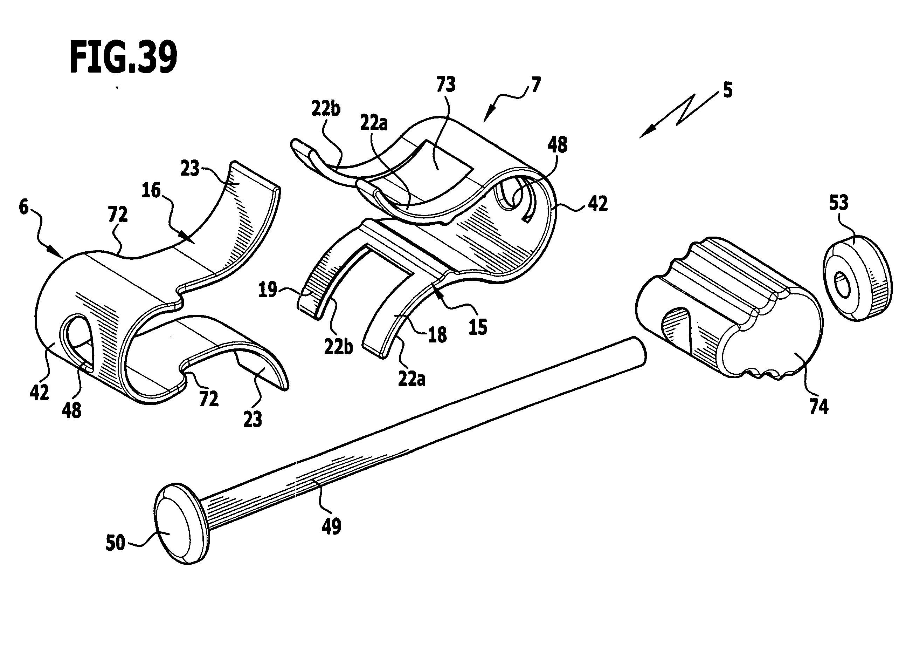

In den Innenraum der Implantatkomponenten, also in den Raum, der von dem Brückenabschnitt 42 umschlossen wird, kann jeweils ein Kern 74 eingelegt werden, wie dies in den Figuren 35 und 36 dargestellt ist. Derartige Kerne können auch bei allen anderen Ausführungsbeispielen Verwendung finden, gegebenenfalls auch nur bei einer der beiden Implantatkomponenten, wie dies in den Figuren 38 und 39 dargestellt ist. Diese Kerne können eine Durchgangsbohrung aufweisen, durch die ein Zuganker 26 hindurchgeführt werden kann, mit dem die beiden Implantatkomponenten 6, 7 in zusammen geschobenem Zustand gegeneinander gespannt werden.

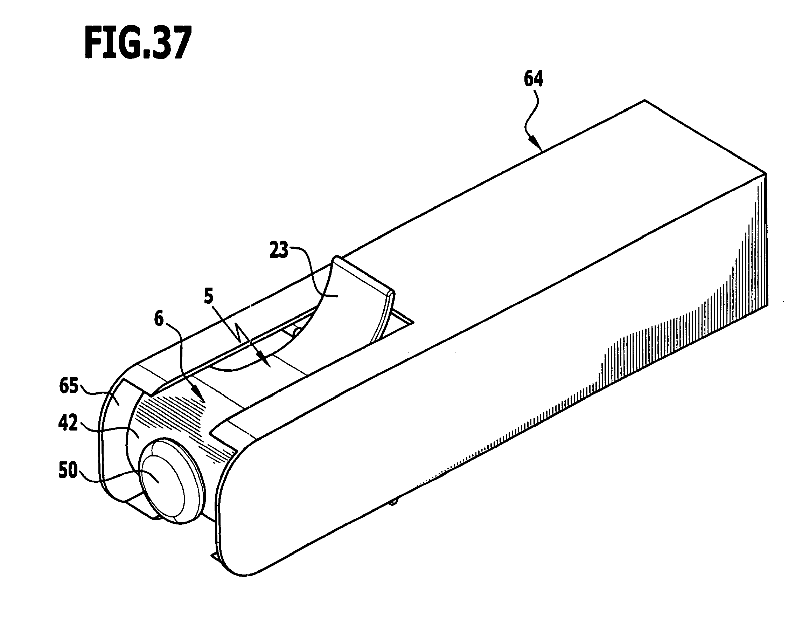

Auch bei diesen Ausführungsbeispielen ist es möglich, das Implantat in einem Gehäuse 64 anzuordnen und dieses nach dem Einsetzen durch eine Öffnung 65 des Gehäuses 64 aus diesem herauszuziehen, in Figur 37 ist das Implantat der Figuren 38 und 39 in einem derartigen Gehäuse 64 dargestellt.

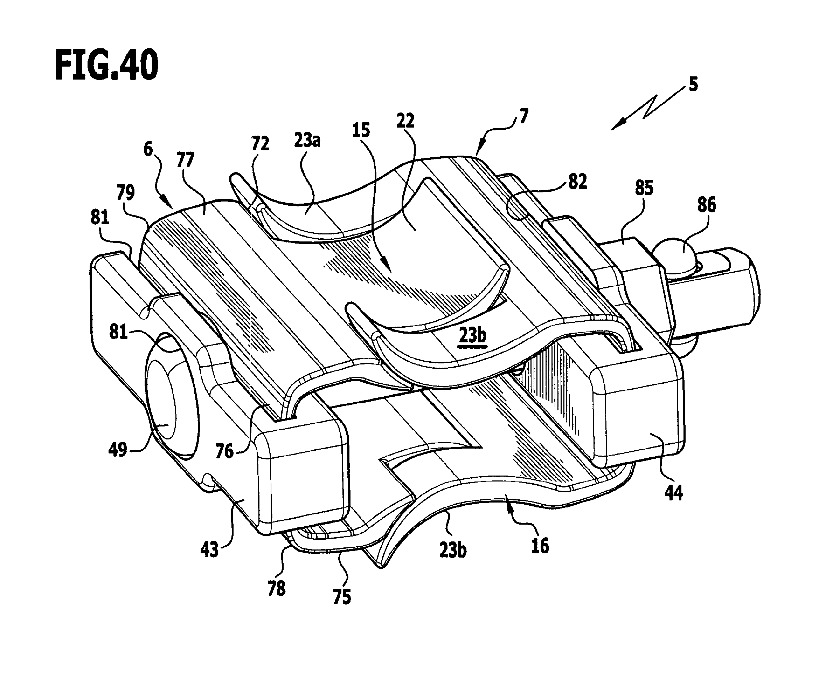

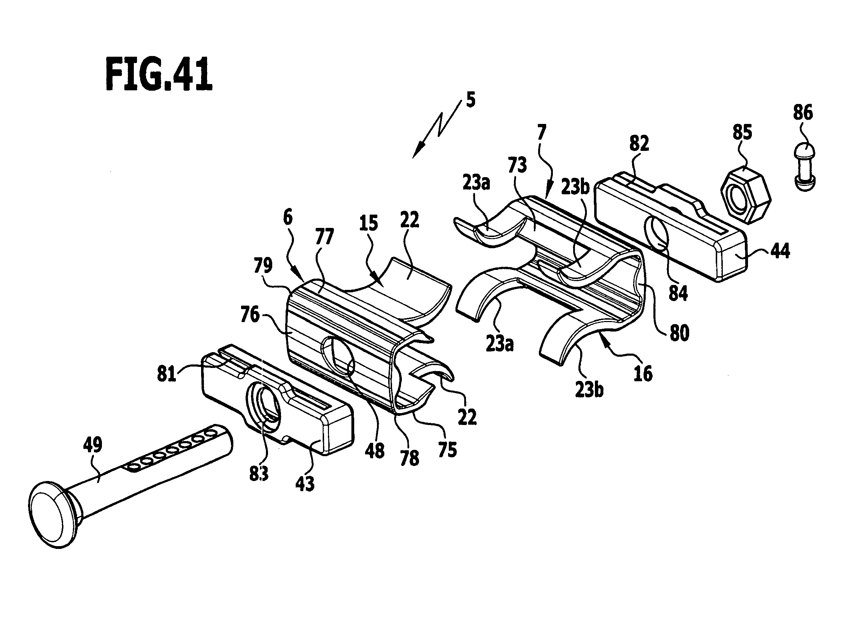

Während bei den bisher beschriebenen Ausführungsformen der Brückenabschnitt 42 im Wesentlichen kontinuierlich gebogen ausgebildet ist oder zweiteilig, können auch andere Querschnittsformen Verwendung finden. Im Ausführungsbeispiel der Figuren 40 und 41 beispielsweise, das ansonsten dem der Figuren 34 und 35 weitgehend entspricht, weist der Brückenabschnitt 3 im Wesentlichen senkrecht zueinander verlaufende ebene Flächen 75, 76, 77 auf, die jeweils über einen abgerundeten Kantenbereich 78, 79 miteinander verbunden sind.

Bei allen Ausführungsformen kann der Brückenabschnitt 42 verstärkt ausgebildet sein, beispielsweise durch eine Vergrößerung der Wandstärke. Bei den Ausführungsbeispielen der Figuren 31 bis 33 sowie 40 und 41 wird dies durch einen Vorsprung 80 an der Innenseite des Brückenabschnittes 42 erreicht. diese Verstärkung der Wand ist besonders dann vorteilhaft, wenn im Brückenabschnitt 42 eine Durchbrechung 48 für einen Zuganker vorgesehen ist, dadurch wird die Stabilität in diesem Bereich trotz dieser Durchbrechung gewährleistet.

Bei dem Ausführungsbeispiel der Figuren 40 und 41 werden die beiden Implantatkomponenten 6, 7 durch einen Zuganker 49 zusammengespannt, der über leistenförmige Endstücke 43, 44 an den Außenseiten der Implantatkomponenten 6, 7 angreift, diese leistenförmigen Endstücke 43, 44 sind dabei gabelförmig ausgebildet mit einem von einer Seite her offenen Längsschlitz 81, 82, sie sind seitlich derart auf die Implantatkomponenten 6, 7 aufgeschoben, dass der Brückenabschnitt 42 in die Längsschlitze 81 beziehungsweise 82 eintaucht, dadurch werden die Endstücke 43 und 44 zuverlässig an den jeweiligen Implantatkomponenten 6, 7 festgelegt. Der Zuganker 49 greift dabei durch Öffnungen 83, 84 in den Endstücken 43 beziehungsweise 44 hindurch, außerdem durch Durchbrechungen 48 in den beiden Implantatkomponenten 6, 7, und nach dem Zusammenspannen wird die Position der Implantatkomponenten 6, 7 dadurch fixiert, dass auf dem Zuganker 49 eine Mutter 85 durch einen den Zuganker 49 durchsetzenden Stift 86 in axialer Richtung gesichert wird.

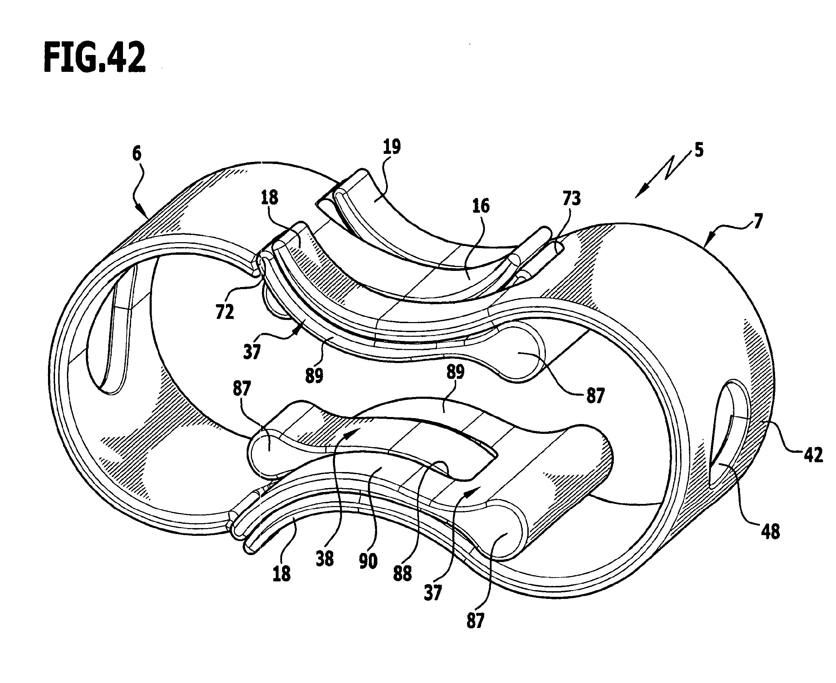

In den Figuren 42 bis 44 ist ein Ausführungsbeispiel eines Implantates beschrieben, das weitgehend dem der Figuren 33 und 34 entspricht. Zusätzlich zu den beiden Implantatkomponenten 6, 7 sind jeweils zwischen die Stützarme der einen Implantatkomponente und die Aufgleitflächen der jeweils anderen Implantatkomponente bandförmige Anlageelemente 37, 38 eingelegt, die ähnlich gebogen sind wie die Stützarme und die zunächst zum Einsetzen eines Implantates an der Innenseite der Stützarme flächig an diesen anliegen. Sie schließen dabei etwa mit dem freien Ende der Stützarme ab und tragen an ihrem gegenüberliegeden Ende eine wurstförmige Verdickung 87. Dabei entsprechen die Anlageelemente in ihren Außenkonturen den Außenkonturen der Stützarme, das heißt an dem Stützarm 16, der durch einen Längsschlitz 17 in zwei parallele Stützarmabschnitte 18, 19 unterteilt ist, liegt ein Anlageelement 37 an, das ebenfalls durch einen Längsschlitz 88 in zwei parallele Abschnitte 89, 90 unterteilt ist, während das andere Anlageelement, welches an dem nicht geteilten Stützarm 15 anliegt, ebenfalls nicht geteilt ist.

Nach dem Einsetzen können die bandförmigen Anlageelemente in ihrer Längsrichtung aus dem Zwischenraum herausgezogen werden, den sie zwischen den Stützarmen 15, 16 einerseits und den Aufgleitflächen 72, 73 andererseits ausfüllen, und dabei richten sie sich mit ihren freien Enden auf, das heißt sie stehen im Wesentlichen quer von der Oberseite der Implantatkomponenten 6, 7 ab, und zwar zu beiden Seiten der Stützflächen 22, 23, die von den Außenseiten der Stützarme in dem anderen freien Ende anschließenden Bereich ausgebildet werden. Durch die wulstförmige Verdickung 87 wird die Ausziehlänge der Anlageelemente begrenzt, so dass es für den Operateur ohne weiteres möglich ist, diese Anlageelemente bis zu ihrem Anschlag herauszuziehen und dadurch das Implantat relativ zum Dornfortsatz in der vorstehend beschriebenen Weise festzulegen.

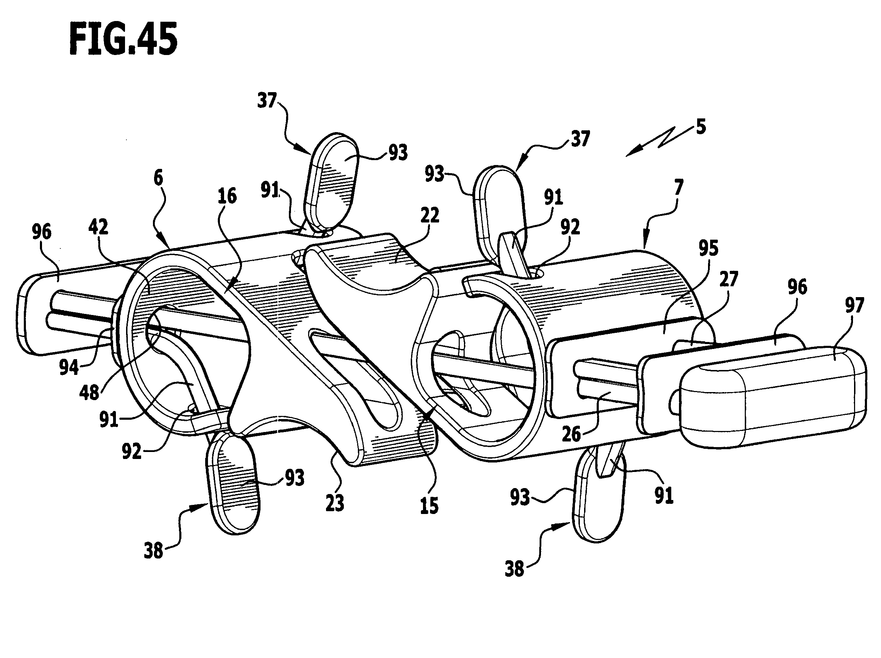

In den Figuren 45 bis 47 ist ein Implantat beschrieben, dessen Implantatkomponente 6, 7 den Implantatkomponenten der Figuren 22 und 23 entsprechen. An Stelle der dort verwendeten Anlageelemente in Form von Schraubenfedern sind jedoch bei dem Ausführungsbeispiel der Figuren 45 bis 47 spezielle Anlageelemente 37, 38 vorgesehen, die im Wesentlichen eine flexible, stabförmige Stütze 91 umfassen, die ausgehend von der Mitte des Brückenabschnittes 42 zunächst in den Innenraum eintreten, und zwar parallel zu der Mittelebene oder Verschiebeebene der Implantatkomponenten, und dann nach einer starken Abwinkelung durch eine Durchbrechung 92 im Endbereich des jeweiligen Brückenabschnittes 42 nach außen austreten. Dabei trägt jede Implantatkomponente 6, 7 zwei derartige Anlageelemente, die nach gegenüberliegenden Seiten aus der Implantatkomponente austreten, alle Stützen 51 tragen an ihrem freien Ende eine vergrößerte Anlagefläche 93.

Die Stützen 91 sind nicht am Brückenabschnitt 42 festgelegt, sondern sie durchsetzen diesen durch Durchbrechungen 48 des Brückenabschnittes 42, durch den auch Zuganker 26 und 27 hindurchgeführt sind. Auf diesen ZuganKern 26, 27 sind außerhalb der Implantatkomponenten 6, 7 auf gegenüberliegenden Seiten derselben zwei Querstege 94, 95 längsverschieblich gelagert, an jedem dieser Querstege 94, 95 sind zwei Anlageelemente 37, 38 mit ihren Stützen 91 festgelegt.

Die beiden Zuganker 26, 27 sind an ihren Enden über zwei brückenförmige Stege 96, 97 verbunden, deren Abstand ist zunächst größer als der Abstand der Implantatkomponenten 6, 7 im zusammengeschobenen Zustand.

Auf diese Weise ist es möglich, die Querstege 94, 95 längs der Zuganker 26, 27 zu verschieben und sie dadurch auch von den Brückenabschnitten 42 zu entfernen. Dies führt dazu, dass die flexiblen Stützen 91 in den Durchbrechungen 48 und 92 verschoben werden, diese Durchbrechungen wirken als Führung bei einer derartigen Verschiebung. Wenn die Stützen 91 durch Annäherung der Querstege 94, 95 an den Brückenabschnitt 42 in Richtung auf die jeweilige Implantatkomponente 6, 7 verschoben werden, führt dies dazu, dass ihre die Anlagefläche 93 tragenden Enden seitlich stärker aus den Implantatkomponenten 6, 7 ausgeschoben werden, und diese ausgeschobenen Stützen mit den Anlageflächen 93 legen sich dann seitlich an die Dornfortsätze an und fixieren auf diese Weise das Implantat relativ zu den Wirbelkörpern.

Wenn also die Implantatkomponenten 6, 7 nach dem Einsetzen durch Verschiebung des Steges 97 auf den Zugankern 26, 27 gegeneinander gespannt werden, führt dies gleichzeitig auch dazu, dass die Querstege 94, 95 gegen die Implantatkomponenten verschoben werden und dass die Stützen 91 der Anlageelemente 37, 38 ausgefahren werden. In diesem Zustand können die Einzelteile durch Festlegung des Steges 97 auf den Zugankern 26, 27 in ihrer erreichten Position fixiert werden.

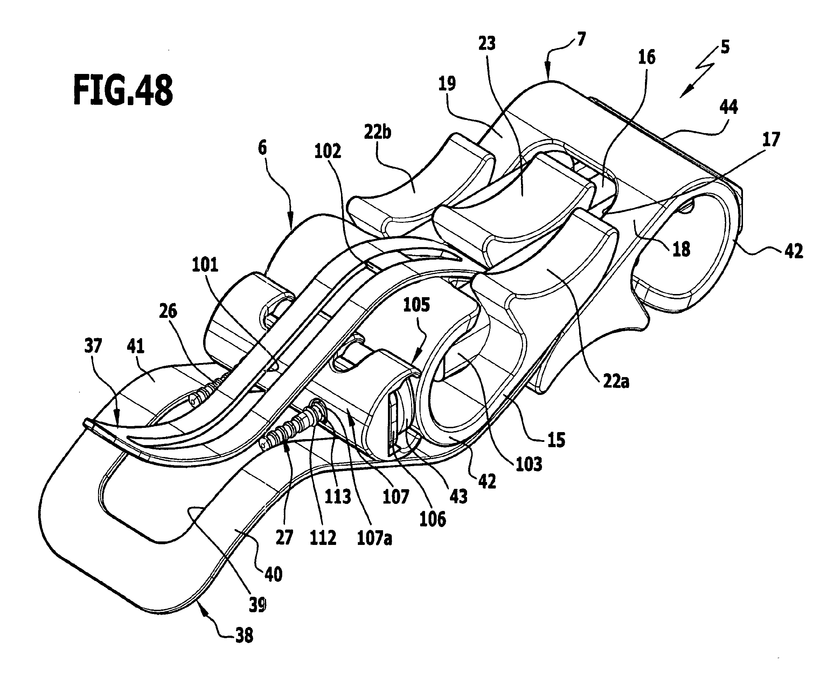

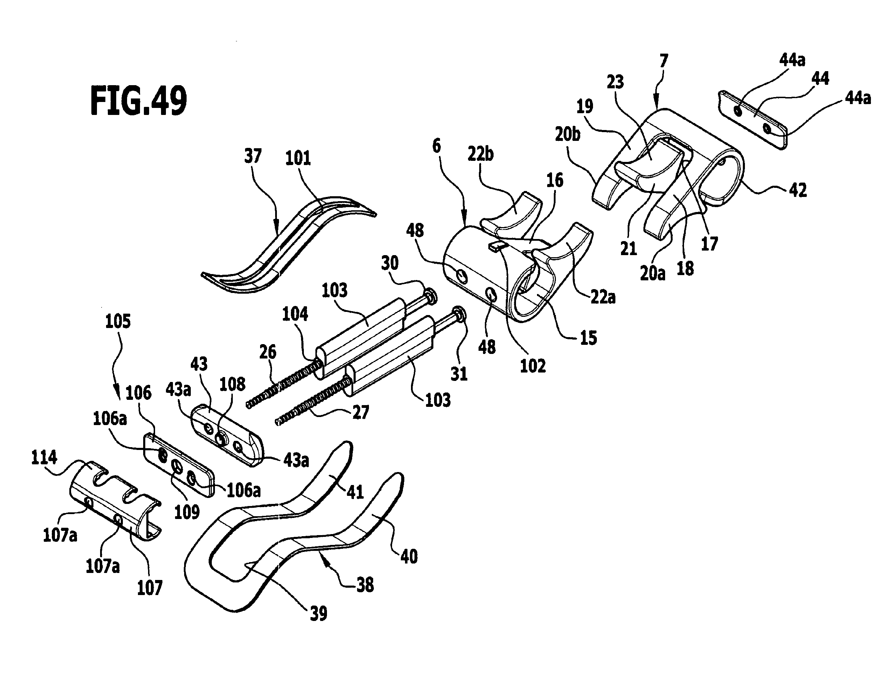

In den Figuren 48 und 49 ist ein weiteres bevorzugtes Ausführungsbeispiel eines Implantates dargestellt. Dieses umfasst Implantatkomponenten 6, 7, die weitgehend denen des Ausführungsbeispieles der Figuren 7 bis 9 entsprechen, einander entsprechende Teile tragen dieselben Bezugszeichen. Auch die Anlageelemente 37 und 38 sind ähnlich aufgebaut wie bei dem Ausführungsbeispiel der Figuren 7 bis 9, auch hier tragen einander entsprechende Teile dieselben Bezugszeichen.

Im Unterschied zu dem Ausführungsbeispiel der Figuren 7 bis 9 weist das bandförmige, schmale Anlageelement 37 einen sich über den größten Teil seiner Länge erstreckenden Längsschlitz 101 auf, in den beim Einschieben des Anlageelementes 37 zwischen die beiden Implantatkomponenten 6, 7 ein Führungsvorsprung 102 eingreift, der an der Außenseite der Implantatkomponente 6 angeordnet ist und der so breit ausgebildet ist, dass er sich an die beiden Seitenkanten des Längsschlitzes 101 anlegt. Dadurch ergibt sich beim Einschieben des Anlageelementes 37 zwischen die beidem Implantatkomponenten 6, 7 eine Führung des Anlageelementes 37, so dass der Benutzer beim Einschieben des Anlageelementes 37 unterstützt wird und eine Vorgabe für die Vorschubrichtung erhält.

Eine ähnliche Führung mittels eines Längsschlitzes und eines Führungsvorsprunges kann auch bei den übrigen Ausführungsbeispielen für das Anlageelement 37 und auch für das Anlageelement 38 erfolgen. Beim Anlageelement 38 ist ein relativ breiter Längsschlitz 39 vorhanden, und an dessen Seitenkanten können sich andere Teile der Implantatkomponenten führend anlegen, beispielsweise zwei die beiden Implantatkomponenten 6, 7 durchsetzende Zuganker 26, 27 oder andere Teile der Implantatkomponenten.

Bei dem Ausführungsbeispiel des Implantates gemäß Figuren 48 und 49 werden die beiden Implantatkomponenten 6, 7 durch zwei nebeneinander angeordnete Zuganker 26, 27 zusammengespannt, die durch Durchbrechungen 48 in den Brückenabschnitten 42 der beiden Implantatkomponenten hindurchgesteckt werden. Diese Zuganker 26, 27 weisen jeweils einen Kopf 30, 31 an einem Ende auf und sind durch Durchbrechungen 44a eines plattenförmigen Endstückes 44 hindurchgesteckt, das an die Außenseite des Brückenabschnittes 42 der Implantatkomponente 7 anlegbar ist. Auf beide Zuganker 26, 27 ist jeweils eine Führungsleiste 103 aufgesteckt, diese Führungsleisten 103 liegen an den Innenkanten des Längsschlitzes 39 des Anlageelementes 38 an und führen dieses bei der Längsverschiebung, das heißt beim Einschieben zwischen die beiden Implantatkomponenten 6, 7. Die Führungsleisten 103 weisen dabei einen in Längsrichtung durchgehenden Aufnahmekanal 104 auf, durch die die Zuganker 26, 27 hindurchgreifen.

Auf der dem Endstück 44 gegenüber liegenden Seite treten die Zuganker 26, 27 durch Durchbrechungen 48 in der Implantatkomponente 6 aus dieser aus, in diesem Bereich ist auf die beiden Zuganker 26, 27 ein ebenfalls plattenförmiges Endstück 43 aufgeschoben, welches nebeneinander zwei Durchbrechungen 43a aufweist, durch die die Zuganker 26, 27 hindurchtreten.

Durch Annäherung der beiden Endstücke 43 und 44 lassen sich die beiden Implantatkomponenten 6, 7 gegeneinander spannen. Um diese Implantatkomponenten in der gegeneinander gespannten Stellung festzulegen, wird eine Verriegelungseinrichtung 105 vorgesehen, die ein plattenförmiges Verriegelungselement 106 und eine auf dieses und das Endstück 43 aufsetzbare Kappe 107 umfasst. Das Endstück 43 trägt zwischen den beiden Durchbrechungen 43a einen zentralen Lagerstutzen 108, der in eine zentrale Lageröffnung 109 der Verriegelungsplatte 106 eingreift, so dass die Verriegelungsplatte106 auf dem plattenförmigen Endstück 43 um die durch den Lagerstutzen 108 gebildete Drehachse verschwenkbar gelagert ist.

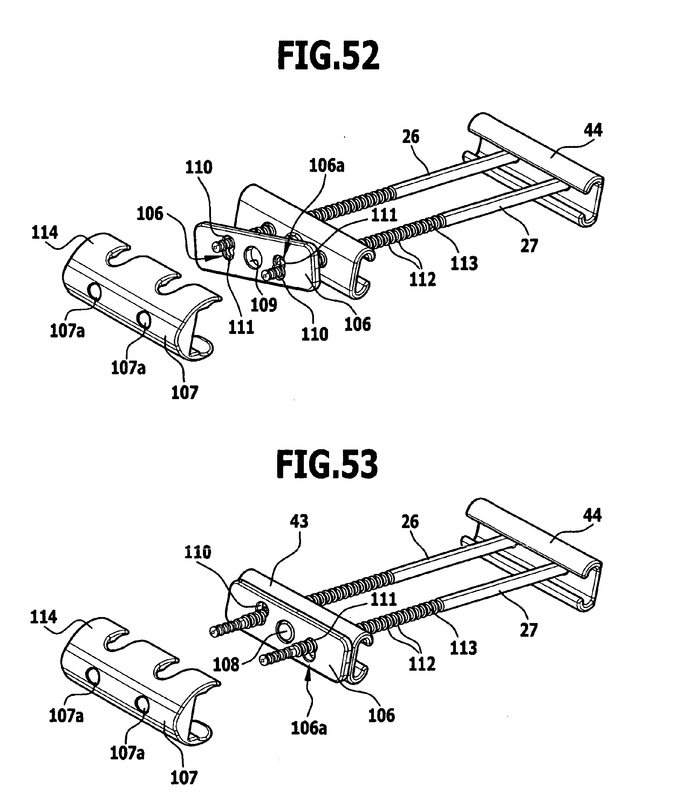

Zu beiden Seiten der Lageröffnung109 sind in der Verriegelungsplatte 106 Durchbrechungen 106a angeordnet, die mit den Durchbrechungen 43a des End-stückes 43 ausgerichtet sind. Wie insbesondere aus den Figuren 52 und 53 deutlich wird, ist die Durchbrechung 106a in zwei nebeneinander liegende Abschnitte unterteilt, nämlich einen ersten Abschnitt 110 mit einem größeren Durchmesser und einen zweiten Abschnitt 111, dessen Durchmesser kleiner ist, zum Beispiel kann im zweiten Abschnitt 111 eine nach innen vorstehende Schulter oder Rippe angeordnet sein, durch die der Durchmesser verkleinert wird.

Die Zuganker 26 und 27 tragen quer zu ihrer Längsrichtung verlaufende, im Abstand zueinander angeordnete Umfangsrippen 112, zwischen denen somit ringnutenförmige Rücksprünge 113 entstehen. Die Durchmesser des ersten Abschnitts 110 und des zweiten Abschnitts 111 sind so gewählt, dass die Zuganker 26, 27 mit ihren Umfangsrippen 112 ohne weiteres in axialer Richtung durch die ersten Abschnitte 110 hindurch geschoben werden können, der Querschnitt der zweiten Abschnitte111 ist aber geringer und erlaubt ein solches freies Hindurchschieben der Umfangsrippen 112 nicht mehr. Dagegen ist der Querschnitt des zweiten Abschnittes 111 so groß, dass die Zuganker 26, 27 mit dem zwischen den Umfangsrippen 112 liegenden Bereich, also mit dem nutförmigen Rücksprung 113 in diesen zweiten Abschnitt 111 eintreten können. Dies führt dazu, dass die neben dem jeweiligen Rücksprung 113 angeordneten Umfangsrippen 112 entweder an beiden Außenflächen der Verriegelungsplatte 106 oder an beiden Seitenflächen einer den Querschnitt des zweiten Abschnittes 111 verkleinernden Schulter oder Rippe anliegen, dadurch werden die Verriegelungsplatte 106 und die beiden Zuganker 26, 27 gegen eine axiale Verschiebung der Zuganker 26, 27 relativ zu der Verriegelungsplatte 106 gesichert.

Durch eine Verschwenkung der Verriegelungsplatte 106 gegenüber dem Endstück 43 und damit gegenüber den durch das Endstück 43 hindurchragenden Zugankern 26, 27 kann damit die Verriegelungsplatte 106 von einer Verriegelungsstellung in eine Freigabestellung und umgekehrt verschwenkt werden, in einer Freigabestellung treten die Zuganker 26, 27 durch den ersten Abschnitt 110 der Verriegelungsplatte 106 hindurch, in der Verriegelungsstellung dagegen durch den zweiten Abschnitt 111. Beim Spannen wird also zunächst die Verriegelungsplatte 106 in der gegenüber dem Endstück 43 verschwenkten Freigabestellung gehalten (Figur 52), in dieser Stellung können die beiden Endstücke 43, 44 ohne weiteres gegen einander gespannt werden, und dies gilt natürlich auch für die an dem Endstück 43 anliegende und durch den Lagerstutzen 108 schwenkbar an diesem Endstück 43 gelagerte Verriegelungsplatte 106. Sobald die beiden Endstücke 43, 44 ausreichend zusammengeschoben sind, wird die Verriegelungsplatte 106 in die in Figur 53 dargestellte Verriegelungsstellung verschwenkt, und dabei treten die Zuganker 26, 27 in den zweiten Abschnitt 111 der Durchbrechungen 106a ein und verhindern somit, dass sich die Verriegelungsplatte 106 und damit auch das Endstück 43 längs der Zuganker 26, 27 verschieben kann.

Sobald diese Verriegelung erreicht ist, wird die Kappe 107 über die Verriegelungsplatte 106 und das Endstück 43 geschoben, wobei die Kappe 107 mit seitlichen Armen 114 sowohl die Verriegelungsplatte 106 als auch das Endstück 43 übergreift. Die Arme 114 sind vorzugsweise elastisch ausgebildet, so dass die Kappe 107 in der aus Figur 48 ersichtlichen Weise elastisch auf dem Endstück 43 und der Verriegelungsplatte 106 gehalten ist. Die Kappe 107 kann dabei zwei Durchbrechungen 107a aufweisen, durch die die Enden der Zuganker 26, 27 hindurchtreten.

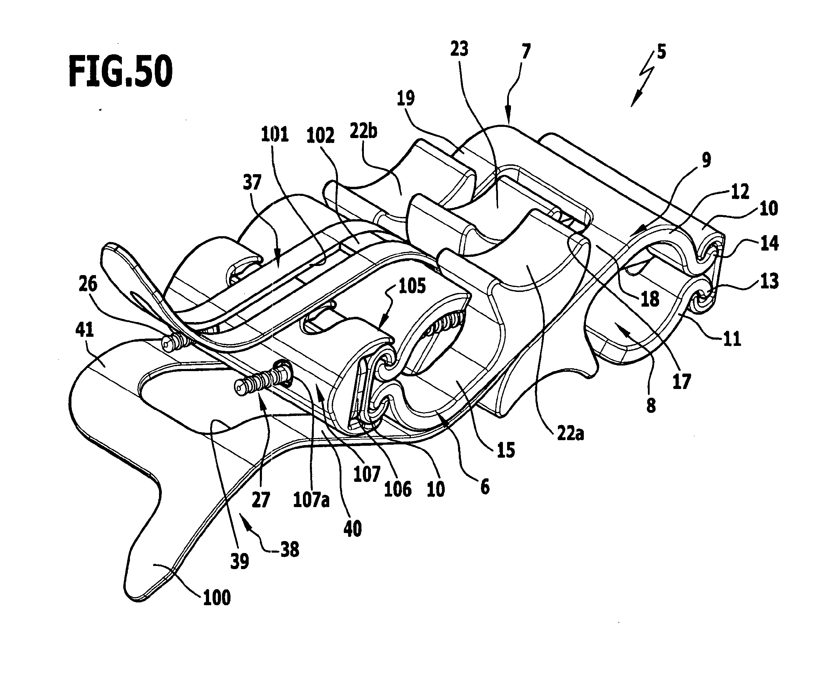

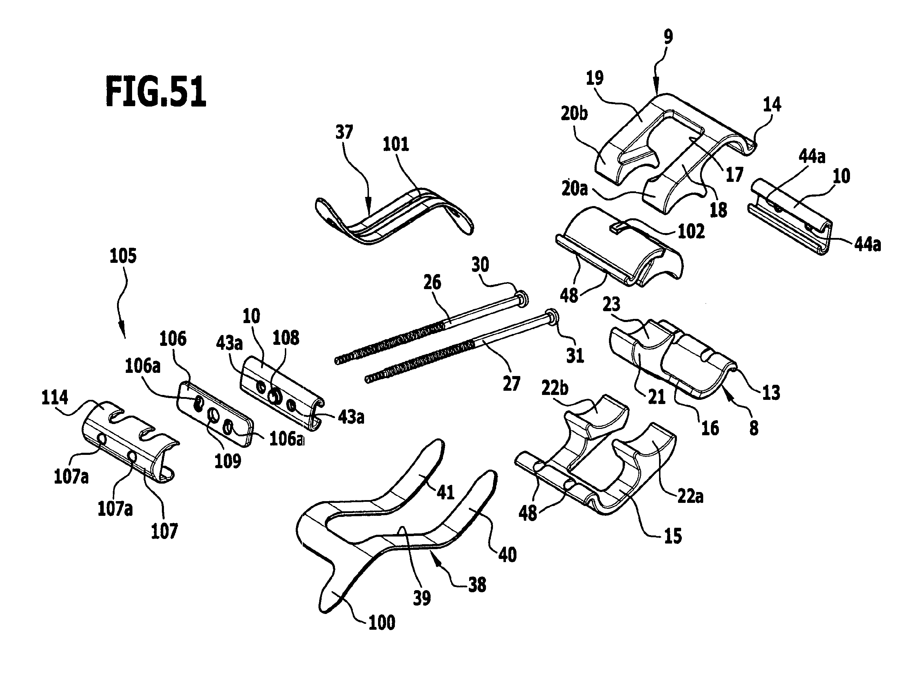

Bei dem Ausführungsbeispiel der Figuren 50 und 51 ist eine insgesamt sehr ähnliche Ausgestaltung gewählt, jedoch sind ähnlich wie bei dem Ausführungsbeispiel der Figuren 3 bis 6 die beiden Implantatkomponenten 6, 7 jeweils aus zwei Bauteilen 8, 9 zusammengesetzt, die in gleicher Weise durch Klammern 10 zusammengehalten werden. Diese Klammern 10 übernehmen damit auch die Aufgabe der Endstücke 43 und 44 und sind abgesehen von ihrer Funktion als Zusammenhalt der Bauteile 8, 9 im wesentlichen gleich aufgebaut wie die Endstücke 43 und 44 bei dem Ausführungsbeispiel der Figuren 48 und 49. Dementsprechend funktioniert auch die Verriegelungseinrichtung 105 in gleicher Weise.

Bei dem Ausführungsbeispiel der Figuren 50 und 51 trägt das Anlageelement 38 an einem seiner Stege 40 eine Verlängerung 100, durch die das Einführen erleichtert wird. Eine solche Verlängerung kann auch bei den anderen Ausführungsbeispielen am Anlageelement 38 vorgesehen werden.

## Patentansprüche

1. Implantat zur gegenseitigen Abstützung der Dornfortsätze (3, 4) von zwei benachbarten Wirbelkörpern (1, 2) mit mindestens einer oberen Stützfläche (23) für den Dornfortsatz (3) eines oberen Wirbelkörpers (1) und mindestens einer unteren Stützfläche (22) für den Dornfortsatz (4) eines unteren Wirbelkörpers (2), mit zwei Implantatkomponenten (6, 7), von denen jede eine Aufgleitfläche (24, 25; 72, 73) aufweist, wobei die Aufgleitflächen (24, 25; 72, 73) der beiden Implantatkomponenten (6, 7) aneinander derart anliegen, dass beim Zusammenschieben der beiden Implantatkomponenten (6, 7) diese Implantatkomponenten (6, 7) längs der Aufgleitflächen (24, 25; 72, 73) aneinander gleiten und dabei der Abstand der oberen Stützfläche (23) von der unteren Stützfläche (22) vergrößert wird, **dadurch gekennzeichnet, dass** jede Implantatkomponente (6, 7) zwei nebeneinander angeordnete, an einem Ende über eine Brücke (10, 11, 12; 42) miteinander verbundene Stützarme (15, 16) aufweist,
dass die beiden Stützarme (15, 16) jeder der beiden Implantatkomponenten (6, 7) an ihren freien Enden auseinanderspreizbar sind,
dass mindestens einer der Stützarme (15, 16) eine der Stützflächen (22, 23) ausbildet,
dass beide Implantatkomponenten (6, 7) mit den freien Enden ihrer Stützarme (15, 16) gegen die freien Enden der Stützarme (16, 15) der jeweils anderen Implantatkomponente (7, 6) gerichtet sind,
dass die Stützarme (15, 16) der einen Implantatkomponente (6, 7) an der Aufgleitfläche (24, 25; 72, 73) der anderen Implantatkomponente (7, 6) anliegen, wobei die Stützarme (15, 16) so angeordnet und geformt sind, dass die Stützarme (15, 16) beim Annähern der beiden Implantatkomponenten (6, 7) an den Aufgleitflächen (24, 25; 72, 73) der jeweils anderen Implantatkomponente (7, 6) aufgleiten und dadurch so verschwenkt werden, dass dadurch der Abstand der oberen und unteren Stützflächen (22, 23) vergrößert wird.

2. Implantat nach Anspruch 1, **dadurch gekennzeichnet, dass** die Stützflächen (22, 23) konkav gekrümmt sind, so dass bei einer Abstützung des Dornfortsatzes (3, 4) im mittleren Teil einer Stützfläche (22, 23) diese mit ihren Randbereichen zu beiden Seiten des Dornfortsatzes (3, 4) ansteigt.

3. Implantat nach einem der voranstehenden Ansprüche, **dadurch gekennzeichnet, dass** jede Implantatkomponente (6, 7) an ihren beiden Stützarmen (15, 16) jeweils eine Stützfläche (22, 23) aufweist, so dass jede Implantatkomponente (6, 7) eine obere und eine untere Stützfläche ausbildet.

4. Implantat nach einem der voranstehenden Ansprüche, **dadurch gekennzeichnet, dass** mindestens ein Stützarm (15) einer Implantatkomponente durch einen an seinem freien Ende beginnenden Längsschlitz (17) in zwei nebeneinander liegende Stützarmabschnitte (18, 19) unterteilt ist.

5. Implantat nach Anspruch 4, **dadurch gekennzeichnet, dass** Stützarme (16, 15) oder Stützarmabschnitte (18, 19) einer Implantatkomponente (6, 7) im Längsschlitz (17) zwischen Stützarmabschnitten (18, 19) der anderen Implantatkomponente (7, 6) angeordnet sind.

6. Implantat nach einem der voranstehenden Ansprüche, **dadurch gekennzeichnet, dass** die Implantatkomponenten (6, 7) im wesentlichen U-förmig ausgebildet sind mit nebeneinander angeordneten Stützarmen (15, 16), die beim Annähern der Implantatkomponenten (6, 7) mit ihren Innenseiten an den Aufgleitflächen (72, 73) der anderen Implantatkomponente (7, 6) aufgleiten und dadurch auseinandergeschwenkt werden.

7. Implantat nach Anspruch 6, **dadurch gekennzeichnet, dass** mindestens ein Stützarm (15, 16) an seiner Außenseite (24, 25) eine Stützfläche (22, 23) trägt.

8. Implantat nach einem der Ansprüche 1 bis 5, **dadurch gekennzeichnet, dass** die Stützarme (15, 16) einer Implantatkomponente (6, 7) ausgehend von der sie verbindenden Brücke (10, 11, 12; 42) so gegeneinander geneigt verlaufen, dass sie sich überkreuzen, und dass die Stützarme (15, 16) mit ihren Außenseiten (24, 25) an den Aufgleitflächen (25, 24) der anderen Implantatkomponente (7, 6) aufgleiten und dadurch noch stärker gegeneinander geneigt werden, wobei sich infolge der Überkreuzung der Stützarme (15, 16) die freien Enden der Stützarme (15, 16) voneinander entfernen.

9. Implantat nach Anspruch 8, **dadurch gekennzeichnet, dass** mindestens ein Stützarm (15, 16) an seiner Innenseite eine Stützfläche (22, 23) trägt.

10. Implantat nach einem der Ansprüche 8 oder 9, **dadurch gekennzeichnet, dass** die Außenseite (24, 25) eines Stützarmes (15, 16) einer Implantatkomponente (6, 7) die Aufgleitfläche für einen Stützarm (15, 16) der anderen Implantatkomponente (7, 6) bildet.

11. Implantat nach einem der voranstehenden Ansprüche, **dadurch gekennzeichnet, dass** beide Implantatkomponenten (6, 7) eines Implantates (5) gleich ausgebildet sind.

12. Implantat nach einem der Ansprüche 1 bis 11, **dadurch gekennzeichnet, dass** eine Implantatkomponente (6, 7) aus zwei Einzelteilen (8, 9) besteht, die im Bereich der Brücke (10, 11, 12) scharnierartig miteinander verbunden sind.

13. Implantat nach Anspruch 12, **dadurch gekennzeichnet, dass** beide Einzelteile (8, 9) an ihren aneinander anliegenden Enden jeweils einen Lagerwulst (13, 14) tragen, die nebeneinander verlaufen und gemeinsam von einer Klammer (10) umfasst und dadurch nebeneinander gehalten werden.

14. Implantat nach einem der voranstehenden Ansprüche, **dadurch gekennzeichnet, dass** die Implantatkomponente (6, 7) im Bereich der Brücke (42) mindestens eine Durchbrechung (48) für einen die beiden Implantatkomponenten (6, 7) gegeneinander schiebenden Zuganker (26, 27; 49) aufweist.

15. Implantat nach einem der voranstehenden Ansprüche, **dadurch gekennzeichnet, dass** durch die Implantatkomponenten (6, 7) mindestens ein Zuganker (26, 27; 49) hindurchgreift, der die beiden Implantatkomponenten (6, 7) gegeneinander spannt.

16. Implantat nach einem der Ansprüche 1 bis 14, **dadurch gekennzeichnet, dass** seitlich neben den Implantatkomponenten (6, 7) einseitig oder beidseitig ein Zuganker (26, 27) angeordnet ist, der außen an den beiden Brücken (10, 11, 12; 42) der beiden Implantatkomponenten (6, 7) anliegende Endstücke (10; 43, 44) aufweist und dadurch die beiden Implantatkomponenten (6, 7) gegeneinander spannt.

## Claims

1. Implant for mutual support of the spinous processes (3, 4) of two adjacent vertebral bodies (1, 2) with at least one upper support surface (23) for the spinous process (3) of an upper vertebral body (1) and at least one lower support surface (22) for the spinous process (4) of a lower vertebral body (2), with two implant components (6, 7), each of which having a slide face (24, 25; 72, 73), the slide faces (24, 25; 72, 73) of the two implant components (6, 7) abutting against one another such that, when pushing the two implant components (6, 7) together, these implant components (6, 7) slide on one another along the slide faces (24, 25; 72, 73) and the spacing of the upper support surface (23) and the lower support surface (22) is thereby increased, **characterised in that** each implant component (6, 7) comprises two adjacent support arms (15, 16), which are connected to one another at one end by means of a bridge (10, 11, 12; 42),
**in that** the two support arms (15, 16) of each of the two implant components (6, 7) can be spread apart at their free ends,
**in that** at least one of the support arms (15, 16) forms one of the support surfaces (22, 23),
**in that** both implant components (6, 7) with the free ends of their support arms (15, 16) are directed towards the free ends of the support arms (16, 15) of the respective other implant component (7, 6),
**in that** the support arms (15, 16) of the one implant component (6, 7) abut against the slide face (24, 25; 72, 73) of the other implant component (7, 6), the support arms (15, 16) being arranged and shaped such that, as the two implant components (6, 7) approach one another, the support arms (15, 16) slide on the slide faces (24, 25; 72, 73) of the respective other implant component (7, 6), and are pivoted thereby, so that the spacing of the upper and lower support surfaces (2, 3) is thereby increased.

2. Implant according to claim 1, wherein the support surfaces (22, 23) are concavely curved, so that when the spinous process (3, 4) is supported in the centre portion of a support surface (22, 23), this rises at its edge regions on both sides of the spinous process (3, 4).

3. Implant according to any of the preceding claims, wherein each implant component (6, 7) has a respective support surface (22, 23) on its two support arms, so that each implant component (6, 7) forms an upper and a lower support surface.

4. Implant according to any of the preceding claims, wherein at least one support arm (15) of an implant component (6, 7) is divided into two adjacent support arm sections (18, 19) by a longitudinal slot (17) starting at its free end.

5. Implant according to claim 4, wherein support arms (16, 15) or support arm sections (18, 19) of one implant component (6, 7) are arranged in the longitudinal slot (17) between support arm sections (18, 19) of the other implant component (7, 6).

6. Implant according to any of the preceding claims, wherein the implant components (6, 7) are configured substantially in a U shape with adjacent support arms (15, 16), which when the implant components (6, 7) approach one another slide with their inner surfaces on the slide faces (72, 73) of the other implant component (7, 6) and are thereby pivoted apart.

7. Implant according to claim 6, wherein at least one support arm (15, 16) bears a support surface (22, 23) on its outer surface (24, 25).

8. Implant according to any one of claims 1 to 5, wherein the support arms (15, 16) of one implant component (6, 7) run inclined relative to one another, starting from the bridge (10, 11, 12; 42) connecting them, that they cross over one another and that the support arms (15, 16) slide on the slide faces (24, 25) of the other implant component (7, 6) with their outer surfaces (24, 25) and are thereby inclined even more steeply relative to one another, wherein as a result of the crossover of the support arms (15, 16) the free ends of the support arms (15, 16) are moved away from one another.

9. Implant according to claim 8, wherein at least one support arm (15, 16) bears a support surface (22, 23) on its inner surface.

10. Implant according to any one of claims 8 or 9, wherein the outer surface (24, 25) of a support arm (15, 16) of one implant component (6, 7) forms the slide face for a support arm (15, 16) of the other implant component (7, 6).

11. Implant according to any of the preceding claims, wherein both implant components (6, 7) of an implant (5) are configured identically.

12. Implant according to any one of claims 1 to 11, wherein an implant component (6, 7) consists of two individual parts (8, 9), which are connected to one another in a hinge-like manner in the region of the bridge (10, 11, 12).

13. Implant according to claim 12, wherein on their ends abutting one another both individual parts (8, 9) respectively bear a locating bead (13, 14), which run next to one another and are jointly embraced by a clamp (10) and are thereby held next to one another.

14. Implant according to any of the preceding claims, wherein in the region of the bridge (42) the implant component (6, 7) has at least one through opening (48) for a tie bar (26, 27; 49) pushing the two implant components (6, 7) towards one another.

15. Implant according to any of the preceding claims, wherein at least one tie bar (26, 27; 49), which clamps the two implant components (6, 7) against one another, engages through the implant components (6, 7).

16. Implant according to any one of claims 1 to 14, wherein there is arranged on one side or on both sides laterally next to the implant components (6, 7), a tie bar (26, 27; 49), which has end pieces (10; 43, 44) abutting externally against the two bridges (10, 11, 12; 42) of the two implant components (6, 7) and thereby clamps the two implant components (6, 7) against one another.

## Revendications

1. Implant pour assurer l'appui réciproque des apophyses épineuses (3, 4) de deux vertèbres (1, 2) voisines, comprenant au moins une surface d'appui supérieure (23) pour l'apophyse épineuse (3) d'une vertèbre supérieure (1) et au moins une surface d'appui inférieure (22) pour l'apophyse épineuse (4) d'une vertèbre inférieure (2), l'implant comprenant deux composants d'implant (6, 7) dont chacun présente une surface de glissement (24, 25; 72, 73), les surfaces de glissement (24, 25; 72, 73) des deux composants d'implant (6, 7) s'appuyant l'une contre l'autre de manière telle, que lors du rapprochement par coulissement des deux composants d'implant (6, 7), ces composants d'implant (6, 7) glissent l'un sur l'autre le long des surfaces de glissement (24, 25; 72, 73) en produisant l'augmentation de la distance de la surface d'appui supérieure (23) à la surface d'appui inférieure (22), **caractérisé en ce que** chaque composant d'implant (6, 7) présente deux bras d'appui (15, 16) agencés côte à côte et reliés à une extrémité par un pontet (10, 11, 12; 42),
**en ce que** les deux bras d'appui (15, 16) de chacun des deux composants d'implant (6, 7) peuvent être écartés à leurs extrémités libres,
**en ce qu'**au moins un des bras d'appui (15, 16) forme l'une des surfaces d'appui (22, 23),
**en ce que** les deux composants d'implant (6, 7) sont dirigés, avec les extrémités libres de leurs bras d'appui (15, 16), contre les extrémités libres des bras d'appui (16, 15) de l'autre composant d'implant respectif (7, 6),
**en ce que** les bras d'appui (15, 16) de l'un des composants d'implant (6, 7) s'appuient sur la surface de glissement (24, 25; 72, 73) de l'autre composant d'implant (7, 6), les bras d'appui (15, 16) étant agencés et configurés de manière à ce que, lors du rapprochement des deux composants d'implant (6, 7), les bras d'appui (15, 16) glissent sur les surfaces de glissement (24, 25; 72, 73) de l'autre composant d'implant respectif (7, 6) en étant ainsi pivotés de façon à augmenter la distance entre les surfaces d'appui supérieure et inférieure (22, 23).

2. Implant selon la revendication 1, **caractérisé en ce que** les surfaces d'appui (22, 23) sont courbées de manière concave, de sorte que lors d'un appui de l'apophyse épineuse (3, 4) dans la partie centrale d'une surface d'appui (22, 23), celle-ci s'élève avec ses zones de bordure de part et d'autre de l'apophyse épineuse (3, 4).

3. Implant selon l'une des revendications précédentes, **caractérisé en ce que** chaque composant d'implant (6, 7) présente sur ses deux bras d'appui (15, 16) respectivement une surface d'appui (22, 23), de sorte que chaque composant d'implant (6, 7) forme une surface d'appui supérieure et inférieure.

4. Implant selon l'une des revendications précédentes, **caractérisé en ce qu'**au moins un bras d'appui (15) d'un composant d'implant est subdivisé, par une fente longitudinale (17) qui débute à son extrémité libre, en deux tronçons de bras d'appui (18, 19) agencés côte à côte.

5. Implant selon la revendication 4, **caractérisé en ce que** des bras d'appui (16, 15) ou des tronçons de bras d'appui (18, 19) d'un composant d'implant (6, 7) sont agencés dans la fente longitudinale (17) entre des tronçons de bras d'appui (18, 19) de l'autre composant d'implant (7, 6).

6. Implant selon l'une des revendications précédentes, **caractérisé en ce que** les composants d'implant (6, 7) sont sensiblement d'une configuration en forme de U avec des bras d'appui (15, 16) agencés côte à côte, qui lors du rapprochement des composants d'implant (6, 7) glissent, avec leurs côtés intérieurs, sur les surfaces de glissement (72, 73) de l'autre composant d'implant (7, 6) en étant ainsi écartés par pivotement.

7. Implant selon la revendication 6, **caractérisé en ce qu'**au moins un bras d'appui (15, 16) porte, sur son côté extérieur (24, 25), une surface d'appui (22, 23).

8. Implant selon l'une des revendications 1 à 5, **caractérisé en ce que** les bras d'appui (15, 16) d'un composant d'implant (6, 7) s'étendent de manière inclinée à partir du pontet (10, 11, 12; 42) qui les relie, de façon à se croiser réciproquement, et **en ce que** les bras d'appui (15, 16) glissent, avec leurs côtés extérieurs (24, 25), sur les surfaces de glissement (25, 24) de l'autre composant d'implant (7, 6) en étant ainsi encore plus fortement inclinés l'un par rapport à l'autre, les extrémités libres des bras d'appui (15, 16) s'éloignant l'une de l'autre en raison du croisement réciproque des bras d'appui (15, 16).

9. Implant selon la revendication 8, **caractérisé en ce qu'**au moins un bras d'appui (15, 16) porte, sur son côté intérieur, une surface d'appui (22, 23).

10. Implant selon l'une des revendications 8 ou 9, **caractérisé en ce que** le côté extérieur (24, 25) d'un bras d'appui (15, 16) d'un composant d'implant (6, 7) forme la surface de glissement pour un bras d'appui (15, 16) de l'autre composant d'implant (7, 6).

11. Implant selon l'une des revendications précédentes, **caractérisé en ce que** les deux composants d'implant (6, 7) d'un implant (5) sont de configuration identique.

12. Implant selon l'une des revendications 1 à 11, **caractérisé en ce qu'**un composant d'implant (6, 7) est constitué de deux parties individuelles (8, 9) qui sont reliées à la manière d'une charnière, dans la zone du pontet (10, 11, 12).

13. Implant selon la revendication 12, **caractérisé en ce que** les deux parties individuelles (8, 9) portent, au niveau de leurs extrémités mutuellement adjacentes, respectivement un bourrelet de palier (13, 14), ces bourrelets de palier s'étendant l'un à côté de l'autre et étant entourés en commun par une agrafe (10) en étant ainsi maintenus mutuellement adjacents.

14. Implant selon l'une des revendications précédentes, **caractérisé en ce que** le composant d'implant (6, 7) présente, dans la zone du pontet (42), au moins un passage (48) pour un tirant (26, 27) permettant de faire coulisser les deux composants d'implant (6, 7) l'un à l'encontre de l'autre.

15. Implant selon l'une des revendications précédentes, **caractérisé en ce qu'**à travers les composants d'implant (6, 7) s'engage au moins un tirant (26, 27; 49), qui serre les deux composants d'implant (6, 7) l'un contre l'autre.

16. Implant selon l'une des revendications 1 à 14, **caractérisé en ce que** latéralement à côté des composants d'implant (6, 7), est agencé, d'un côté ou des deux côtés, un tirant (26, 27), qui présente à l'extérieur, des pièces d'extrémité (10; 43, 44) en appui sur les deux pontets (10, 11, 12; 42) des deux composants d'implant (6, 7), en serrant ainsi les deux composants d'implant (6, 7) l'un contre l'autre.
